(19) Europäisches Patentamt
European Patent Office
Office européen des brevets

(11) Publication number: 0 155 623
A2

(12) # EUROPEAN PATENT APPLICATION

(21) Application number: 85102786.2

(22) Date of filing: 12.03.85

(51) Int. Cl.⁴: A 61 K 31/535
A 61 K 31/54, A 61 K 31/495
//C07D279/20, C07D279/36,
C07D279/32, C07D279/34

(30) Priority: 19.03.84 US 591132
21.12.84 US 685101

(43) Date of publication of application:
25.09.85 Bulletin 85/39

(84) Designated Contracting States:
CH DE FR GB IT LI NL

(71) Applicant: MERCK & CO. INC.
126, East Lincoln Avenue P.O. Box 2000
Rahway New Jersey 07065(US)

(72) Inventor: Goldenberg, Marvin M.
721 Shackamaxon Drive
Westfield, N.J. 07090(US)

(74) Representative: Blum, Rudolf Emil Ernst et al,
c/o E. Blum & Co Patentanwälte Vorderberg 11
CH-8044 Zürich(CH)

(54) **Use of lipoxygenase inhibitors for the preparations of cytoprotective pharmaceutical compostions and process for the preparation of a cytoprotective pharmaceutical composition.**

(57) Lipoxygenase inhibitors are used as cytoprotective active ingredient in producing cytoprotective pharmaceutical compositions. Corresponding cytoprotective pharmaceutical compositions are prepared by incorporating into a pharmaceutical composition a lipoxygenase inhibitor in a cytoprotective active amount. The prepared cytoprotective therapeutic composition may be used to treat or prevent disease states such as erosive gastritis, erosive esophagitis, inflammatory bowel disease, and induced hemorrhagic gastric erosions such as those caused by indomethacin or ethanol.

The lipoxygenase inhibitors induce cytoprotection via an unspecified mechanism.

EP 0 155 623 A2

17055Y

TITLE OF THE INVENTION

Use of LIPOXYGENASE INHIBITORS for the preparation of CYTOPROTECTIVE pharmaceutical compositions and process for the preparation of a cytoprotective pharmaceutical composition.

BACKGROUND OF THE INVENTION

The present invention concerns the use of LIPOXYGENASE INHIBITORS for the preparation of cytoprotective pharmaceutical compositions which contain as cytoprotective active component at least one lipoxygenase inhibitor.

The present invention, furthermore, concerns a process for the preparation of a cytoprotective pharmaceutical composition which contains as cytoprotective active component at least one lipoxygenase inhibitor.

The cytoprotective pharmaceutical compositions which are produced according to the inventive use of at least lipoxygenase inhibitor or which are produced according to the inventive process by incorporating into said cytoprotective compositions as cytoprotective active component at least one lipoxygenase inhibitor, can be administered to a mammal, specially to man, for inducing cytoprotection. The pharmaceutical compositions accordingly are useful as inhibitors of mammalian leukotriene biosynthesis.

The lipoxygenase inhibitors which are used according to the present invention are prior art pharmaceutically active ingredients which were used in corresponding pharmaceutical compositions for treating allergic conditions, asthma, cardiovascular disorders, and inflammations.

Prior to this invention, there was no evidence in the literature that the lipoxygenase inhibitors were cytoprotective.

SUMMARY OF THE INVENTION

The present invention concerns the use of lipoxygenase inhibitors for the preparation of cytoprotective pharmaceutical compositions which contain as cytoprotective active component at least one lipoxygenase inhibitor.

The present invention, furthermore, concerns a process for the preparation of a cytoprotective pharmaceutical composition in which there is used as cytoprotective active component at least one lipoxygenase inhibitor.

Preferred lipoxygenase inhibitors which are used according to the present invention are the compounds of formula I:

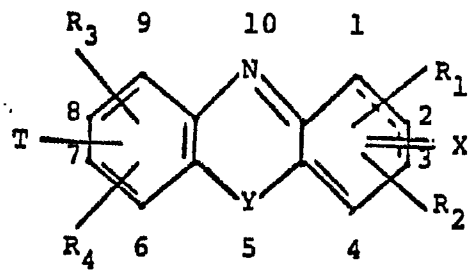

I

or pharmaceutically acceptable salts of the compounds of formula I.

According to a preferred embodiment of the invention, at least one lipoxygenase inhibitor is used for the preparation of a cytoprotective pharmaceutical composition which contains as cytoprotective active ingredient at least one lipogenase inhibitor and, furthermore, at least one further biologically active ingredient, for example a further cytoprotective active ingredient and/or a non-steroidal anti-inflammatory drug.

DETAILED DESCRIPTION OF THE INVENTION

The present invention is directed to the use of lipoxygenase inhibitors generally, and preferably compounds of formula I, as cytoprotective active components for producing cytoprotective active pharmaceutical compositions. The so produced cytoprotective pharmaceutical compositions may be used to treat or prevent disease states such as erosive gastritis; erosive esophagitis; inflammatory bowel disease; ethanol-induced hemorrhagic erosions; hepatic

ischemia; noxious agent induced damage or necrosis of hepatic, pancreatic, renal, or myocardial tissue; liver parenchymal damage caused by hepatoxic agents such as

$CCl_4$ and D-galactosamine; ischemic renal failure; disease-induced hepatic damage; bile salt induced pancreatic or gastric damage; trauma- or stress-induced cell damage; and glycerol-induced renal failure.

The cytoprotective activity of a compound may be observed in both animals and man by noting the increased resistance of the gastrointestinal mucosa to the noxious effects of strong irritants, for example, the ulcerogenic effects of aspirin or indomethacin. In addition to lessening the effect of non-steroidal anti-inflammatory drugs on the gastrointestinal tract, animal studies show that cytoprotective compounds will prevent gastric lesions induced by oral administration of strong acids, strong bases, ethanol, hypertonic saline solutions and the like.

Two assays can be used to measure cytoprotective ability. These assays are; (A) an ethanol-induced lesion assay and (B) an indomethacin-induced ulcer assay.

A. Ethanol-Induced Lesion Assay

Twenty-four hour fasted Sprague-Dawley (S.D.) rats are perorally (p.o.) dosed with 1.0 ml absolute ethanol. Fifteen min. prior to ethanol administration, groups of rats each receive either an aqueous vehicle (aqueous methylcellulose 5% wt.) or the test compound at various doses perorally. One hour later, the animals are sacrificed and stomach ucosae are examined for resulting lesions.

B. Indomethacin-Induced Ulcer Assay

Indomethacin, 10 mg/kg p.o., is used to induce ulcers in 24 hr. fasted S.D. rats. Fifteen

minutes prior to indomethacin administration, groups of rats each receive either an aqueous vehicle (5% wt. methylcellulose) or the test compound at various doses perorally. Four hours later the animals are sacrificed and stomach mucosae are examined for resulting ulcers.

As cytoprotective agents, the lipoxygenase inhibitors may generally be administered at a dosage range of 0.1 µg/kg to 500 mg/kg of body weight. The exact amount of inhibitor to be used will depend on, inter alia, whether it is being administered to heal damaged cells or to avoid future damage, on the nature of the damaged cells (e.g., gastro-intestinal ulcerations vs. nephrotic necrosis), and on the nature of the causative agent.

The effective daily dosage level for compounds of Formula I inducing cytoprotection in mammals, especially humans, will range from about 1 µg/kg to about 50 mg/kg, preferably from about 10 µg/kg to about 10 mg/kg. The dosage may be administered in single or divided individual doses.

Any suitable route of administration may be employed for providing a mammal, especially a human, with a cytoprotectively effective dosage of a lipoxygenase inhibitor. For example, oral, rectal, transdermal, parenteral, intramuscular, intraveneous and the like may be employed. Dosage forms include tablets, troches, dispersions, suspensions, solutions, capsules and the like.

In practical use, lipoxygenase inhibitors can be combined as the active ingredient in intimate admixture with a pharmaceutical carrier according to conventional pharmaceutical compounding techniques.

The carrier may take a wide variety of forms depending on the form of preparation desired for administration, e.g., oral or intravenous. In preparing the compositions for oral dosage form, any of the usual pharmaceutical media may be employed, such as, for example, water glycols, oils, alcohols, flavoring agents, preservatives, coloring agents and the like in the case of oral liquid preparations, such as, for example, suspensions, elixirs and solutions; or carriers such as starches, sugars, diluents, granulating agents, lubricants, binders, disintegrating agents and the like in the case of oral solid preparations such as, for example, powders, capsules and tablets. Because of their ease of administration, tablets and capsules represent the most advantageous oral dosage unit form, in which case solid pharmaceutical carriers are obviously employed. If desired, tablets may be sugar coated or enteric coated by standard techniques.

In addition to the common dosage forms set out above, the lipoxygenase inhibitors may also be administered by controlled release means and/or delivery devices such as those described in U.S. Patent Nos. 3,845,770; 3,916,899; 3,536,809; 3,598,123; 3,630,200 and 4,008,719.

In preparing suitable dosage forms, conventional compounding procedures and ingredients e.g. diluents, carriers, etc. may be used. The following are examples of representative pharmaceutical dosage forms fo Formula I compounds:

| Injectable Suspension | mg/mL |
|---|---|
| Compound of Formula I | 0.7-350 |
| Methylcellulose | 5.0 |
| Tween 80 | 0.5 |
| Benzyl alcohol | 9.0 |
| Methyl paraben | 1.8 |
| Propyl paraben | 0.2 |

Water for injection to a total volume of 1 ml

| Tablet | mg/tablet |
|---|---|
| Compound of Formula I | 0.7-350 |
| Microcrystalline Cellulose | 0-349.8 |
| Providone | 14.0 |
| Microcrystalline Cellulose | 90.0 |
| Pregelatinized Starch | 43.5 |
| Magnesium Stearate | 2-2.5 |
| | 500 |

| Capsule | mg/capsule |
|---|---|
| Compound of Formula I | 0.7-350 |
| Lactose Powder | 248.5-598.3 |
| Magnesium Stearate | 1-1.5 |
| | 600 |

In addition to the lipoxygenase inhibitors, the pharmaceutical compositions can also contain other active ingredients, such as cyclooxygenase inhibitors or non-steroidal anti-inflammatory drugs (NSAIDs). NSAIDs can be characterized into five groups:

(1) the propionic acid derivatives;

(2) the acetic acid derivatives;

(3) the fenamic acid derivatives;

(4) the biphenylcarboxylic acid derivatives; and

(5) the oxicams

or a pharmaceutically acceptable salt thereof.

The propionic acid derivatives which may be used comprise: ibuprofen, ibuprufen aluminum, indoprufen, ketoprufen, naproxen, benoxaprofen, flurbiprofen, fenoprofen, fenbufen, ketoprofen, indoprofen, pirprofen, carprofen, oxaprozin, pranoprofen, miroprofen, tioxaprofen, suprofen, alminoprofen, tiaprofenic acid, fluprofen and bucloxic acid. Structurally related propionic acid derivatives having similar analgesic and anti-inflammatory properties are also intended to be included in this group.

Thus, "propionic acid derivatives" as defined herein are non-narcotic analgesics/non-steroidal anti-inflammatory drugs having a free $-CH(CH_3)COOH$ or $-CH_2CH_2COOH$ group (which optionally can be in the form of a pharmaceutically acceptable salt group, e.g., $-CH(CH_3)COO^-Na^+$ or $-CH_2CH_2COO^-Na^+$), typically attached directly or via a carbonyl function to a ring system, preferably to an aromatic ring system.

The acetic acid derivatives which may be used comprise: indomethacin, sulindac, tolmetin, zomepirac, diclofenac, fenclofenac, alclofenac, ibufenac, isoxepac, furofenac, tiopinac, zidometacin, acemetacin, fentiazac, clidanac, oxpinac, and fenclozic acid. Structually related acetic acid derivatives having similar analgesic and anti-inflammatory properties are also intended to be encompassed by this group.

Thus, "acetic acid derivatives" as defined herein are non-narcotic analgesics/non-steroidal anti-inflammatory drugs having a free $-CH_2COOH$ group (which optionally can be in the form of a pharmaceutically acceptable salt group, e.g. $-CH_2COO^-Na^+$), typically attached directly to a ring system, preferably to an aromatic or heteroaromatic ring system.

The fenamic acid derivatives which may be used comprise: mefenamic acid, meclofenamic acid, flufenamic acid, niflumic acid and tolfenamic acid. Structurally related fenamic acid derivatives having similar analgesic and anti-inflammatory properties are also intended to be encompassed by this group.

Thus, "fenamic acid derivatives" as defined herein are non-narcotic analgesics/non-steroidal anti-inflammatory drugs which contain the basic structure:

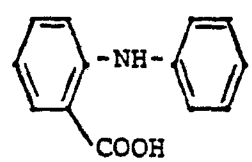

which can bear a variety of substituents and in which the free -COOH group can be in the form of a pharmaceutically acceptable salt group, e.g., $-COO^-Na^+$.

The biphenylcarboxylic acid derivatives which can be used comprise: diflunisal and flufenisal. Structurally related biphenylcarboxylic acid derivatives having similar analgesic and anti-inflammatory properties are also intended to be encompassed by this group.

Thus, "biphenylcarboxylic acid derivatives" as defined herein are non-narcotic analgesics/non-steroidal anti-inflammatory drugs which contain the basic structure:

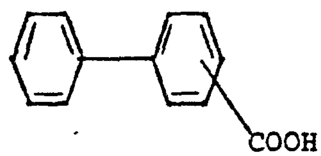

which can bear a variety of substituents and in which the free -COOH group can be in the form of a pharmaceutically acceptable salt group, e.g., $-COO^{-}Na^{+}$.

The oxicams which can be used in the present invention comprise: piroxicam, sudoxicam, isoxicam and 4-hydroxyl-1,2-benzothiazine 1,1-dioxide 4-(N-phenyl)-carboxamide. Structurally related oxicams having similar analgesic and anti-inflammatory properties are also intended to be encompassed by this group.

Thus, "oxicams" as defined herein are non-narcotic analgesics/non-steroidal anti-inflammatory drugs which have the general formula:

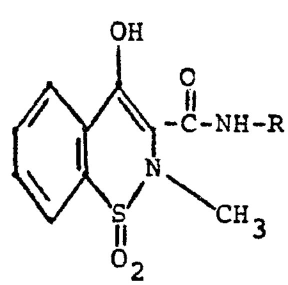

wherein R is an aryl or heteroaryl ring system.

The following NSAIDs may also be used: acemetacin, alminoprofen, amfenac sodium, aminoprofen, anitrazafen, antrafenine, auranofin, bendazac lysinate, benzydamine, beprozin, broperamole, bufezolac, carprofen, cinmetacin, ciproquazone, clidanac, cloximate, dazidamine, deboxamet, delmetacin, detomidine, dexindoprofen, diacerein, di-fisalamine, difenpyramide, emorfazone, enfenamic acid, enolicam, epirizole, etersalate, etodolac, etofenamate, fanetizole mesylate, fenclofenac, fenclorac, fendosal, fenflumizole, fentiazac, feprazone, floctafenine, flunixin, flunoxaprofen, fluproquazone, fopirtoline, fosfosal, furcloprofen, furofenac, glucametacin, guaimesal, ibuproxam, isofezolac, isonixim, isoprofen, isoxepac, isoxicam, lefetamine HCl, leflunomide, lofemizole, lonazolac calcium, lotifazole, loxoprofen, lysin clonixinate, meclofenamate sodium, meseclazone, miroprofen, nabumetone, nictindole, nimesulide, orpanoxin, oxametacin, oxapadol, oxaprozin, perisoxal citrate, pimeprofen, pimetacin, piproxen, pirazolac, pirfenidone, pirprofen, pranoprofen, proglumetacin maleate, proquazone, pyridoxiprofen, sudoxicam, suprofen, talmetacin, talniflumate, tenoxicam, thiazolinobutazone, thielavin B, tiaprofenic acid, tiaramide HCl, tiflamizole, timegadine, tioxaprofen, tolfenamic acid, tolpadol, tryptamid, ufenamate, and zidometacin.

The following NSAIDs, designated by company code number, may also be used: 480156S, AA861, AD1491, AD1590, AFP802, AFP860, AHR6293, AI77B, AP504, AU8001, BAYo8276, BPPC, BW540C, BW755C, CHINOIN 127, CN100, CO893XX, CPP, D10242, DKA9, DV17, EB382, EGYT2829, EL508, F1044,

FZ, GP53633, GP650, GV3658, HG/3, ITC1, ITF, ITF182, KB1043, KC8973, KCNTEI6090, KME4, LA2851, LT696, LU20884, M7074, MED15, MG18311, MR714, MR897, MY309, NO164, ONO3144, PR823, PV102, PV108, QZ16, R830, RS2131, RU16029, RU26559, RUB265, SCR152, SH440, SIR133, SIR136, SIR92, SPAS510, SQ27239, ST281, SX1032, SY6001, SaH46798, TA60, TAI901, TEI615, TVX2706, TVX960, TZI615, U60257, UR2310, WY23205, WY41770, YM09561, YM13162, YS1033, and ZK31945.

Finally, NSAIDs which may also be used include the salicylates, specifically aspirin, and the phenylbutazones, and pharmaceutically acceptable salts thereof.

The lipoxygenase inhibitors may also be used in combination with leukotriene antagonists such as those disclosed in copending applications U.S. Serial Nos. 520,051 and 520,052, filed August 5, 1983 which are herein incorporated by reference and others known in the art such as those disclosed in European Patent Application Nos. 56,172 and 61,800; and in U.K. Patent Specification No. 2,058,785. These pharmaceutical compositions may also contain as the second active ingredient, antihistaminic agents such as benadryl, dramamine, histadyl, phenergan and the like. Alternatively, they may include prostaglandin antagonists such as those disclosed in European Patent Application 11,067 or thromboxane antagonists such as those disclosed in U.S. 4,237,160. They may also contain histidine decarboxyase inhibitors such

as α-fluoromethylhistidine, described in U.S. 4,325,961. The lipoxygenase inhibitors may also be advantageously combined with an $H_1$ or $H_2$-receptor antagonist, such as for instance cimetidine, ranitidine, terfenadine, famotidine, aminothiadiazoles disclosed in EP 81102976.8 and like compounds, such as those disclosed in U.S. Patent Nos. 4,283,408; 4,362,736; 4,394,508; European Patent Application No. 40,696 and a pending application, U.S.S.N. 301,616, filed September 14, 1981. Each of these references is herein incorporated by reference. The pharmaceutical compositions may also contain a $K^+/H^+$ ATPase inhibitor such as omeprazole, disclosed in U.S. Pat. 4,255,431, and the like. The weight ratio of the lipoxygenase inhibitor to NSAID or other active ingredient may be varied and may range from 1000:1 to 1:1000, preferably from 200:1 to 1:200, an effective amount of each compound being used.

A further embodiment of this invention is a method of reducing the undesirable side effects of NSAIDs which comprises the concomitant administration of a cytoprotectively effective amount of a lipoxygenase inhibitor, especially one of Formula I, and an NSAID. By concomitant is meant that the lipoxygenase inhibitor is administered from 30 minutes prior up to 30 minutes after administration of the NSAID. Preferably, the lipoxygenase inhibitor is administered prior to or simultaneously with the NSAID.

In the compositions and methods of this invention, the compound 4-bromo-2,7-dimethoxy-3H-phenothiazin-3-one is particularly preferred.

Specific compounds useful in this invention are those of Formula I:

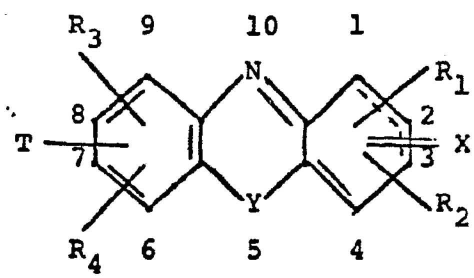

I

wherein

X is in the 1 or 3 position and is O, S or NR wherein R is H, $C_1$-$C_6$ branched or linear alkyl, CN or phenyl;
Y is O, Se, S, SO, $SO_2$ or NR; and the broken line represents an optional double bond between the 1 and 2 or 2 and 3 position;
$R_1$, $R_2$, $R_3$ and $R_4$ are independently selected from:

(1) hydrogen;
(2) alkyl having 1-6 carbon atoms;
(3) alkenyl having 2-6 carbon atoms;
(4) $-(CH_2)_nM$
wherein n is 0-6 and M is
a) $OR_5$;
b) halogen;
c) $CF_3$;

d) $SR_5$ wherein $R_5$ is H; lower alkoxy-lower alkyl; lower acyloxy-lower alkyl; $C_1$-$C_6$ alkyl; benzyl; phenyl or substituted phenyl wherein the substituents are $C_1$-$C_3$ alkyl, halogen, CN, $CF_3$, $COOR_6$, $CH_2COOR_6$, $(CH_2)_nNR_8R_9$ wherein n is 0 to 2, $C_1$-$C_3$ alkoxy, OH, halo-$C_1$-$C_6$-alkyl; $-(CH_2)_mCOOR_6$, wherein m is 0 to 6 and $R_6$ is H, phenyl, or $C_1$-$C_6$ alkyl; CN; formyl; perfluoroalkyl; or $CH_2$-$R_{12}$ wherein n is 0 to 4, $R_{12}$ is $C_1$-$C_5$ alkyl, dimethylamino or phenyl;

(e) phenyl or substituted phenyl as defined above for $R_5$;

(f) $COOR_6$;

(g) $-C(=O)-R_{14}$ wherein $R_{14}$ is H, $(CH_2)_nCOOR_6$ wherein n is 0 to 4, $C_1$-$C_6$ alkyl, $CF_3$, phenyl, or substituted phenyl as defined above for $R_5$;

(h) tetrazole;

(i) $-NH-\overset{O}{\overset{\|}{C}}-R_7$ wherein $R_7$ is $C_1$-$C_6$ alkyl, benzyl or phenyl;

(j) $-NR_8R_9$ wherein $R_8$ and $R_9$ are independently selected from H, phenyl or substituted phenyl as defined above for $R_5$, $C_1$-$C_4$ alkyl, $C_1$-$C_4$ alkylamino alkyl, or may be joined through the N to form a heterocycloalkyl of 5-8 ring atoms;

(k) $-NHSO_2R_{10}$ wherein $R_{10}$ is OH, $C_1$-$C_6$ alkyl, $C_1$-$C_6$-alkoxy, phenyl, or $CF_3$;

(l) $-\overset{O}{\overset{\|}{C}}-CH_2OH$;

(m) $-SOR_{11}$ wherein $R_{11}$ is $C_1$-$C_6$ alkyl, phenyl or substituted phenyl as defined above for $R_5$, $(CH_2)_mCOOR_6$ wherein m is 1 to 6, CN, formyl or perfluoro-$C_1$-$C_4$ alkyl;

(n) $-CONR_8R_9$;

(o) $-SO_2NR_8R_9$;

(p) $-SO_2R_{13}$ wherein $R_{13}$ is OH, $C_1$-$C_6$ alkyl, H, phenyl or substituted phenyl as defined above for $R_5$,

$(CH_2)_mCOOR_6$ wherein m is 1 to 6, CN, formyl or perfluoro-$C_1$-$C_4$ alkyl;

(q) $NO_2$ ;

(r) $O-\overset{O}{\overset{\|}{C}}-R_{14}$ ;

(s) $O-\overset{O}{\overset{\|}{C}}-NR_8R_9$

(t) $O-\overset{O}{\overset{\|}{C}}-OR_7$ ;

(u) CN;

(v) $NR_{15}$ $R_{16}$ wherein $R_{15}$ and $R_{16}$ aare such that $HNR_{15}R_{16}$ is an essential amino acid; or

(5) any two of $R_1$, $R_2$, $R_3$ and $R_4$ may be fused to add a fourth ring, which may be saturated or unsaturated, of five or si^ carbons; and

T is H, halogen or $CF_3$.

The numbers surrounding Formula I designate the substituent positions. T, $R_1$, $R_2$, $R_3$ and $R_4$ may be positioned anywhere in the structure. $R_1$, $R_2$, $R_3$ and $R_4$ may be joined, e.g. as $-(CH_2)_{3-4}-$ to add a fourth ring to the basic three ring structure. This fourth ring may have five or six carbon atoms and may be saturated or unsaturated. For example, compounds of Formula II may be prepared by linking two of the substituent groups; $R_1$, $R_2$, $R_3$, $R_4$:

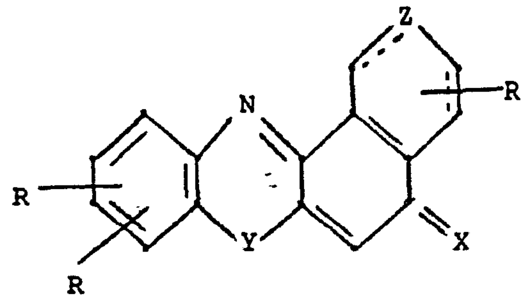

II

wherein Z may be CH, $CH_2$ or a bond, the broken lines represent optional double bonds and R represents the substituent groups of Formula I ($R_1$, $R_2$, $R_3$, $R_4$ and/or T) not used to create the fourth ring.

The preferred compounds of Formula II have the structure II(a):

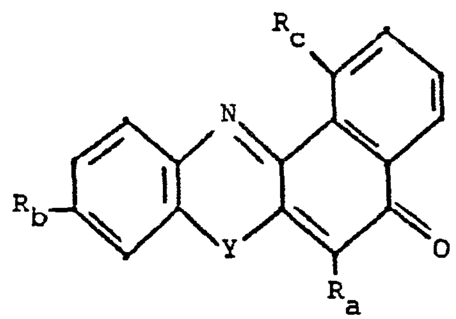

II(a)

wherein $R_a$ is selected from hydrogen, halogen (F, Br, Cl, I), $CH_3$, $CF_3$, $COR_d$, $NHR_d$, $SR_d$ and $OR_d$; $R_b$ is selected from hydrogen, halogen (F, Br, Cl, I), $CH_3$, $CF_3$, $CH_2OH$, $OR_d$, $SR_d$, $COR_d$, $COOR_d$, $CH_2COOR_d$ and $CH(CH_3)COOR_d$; $R_d$ is hydrogen, phenyl, $C_{1-4}$ straight or branched alkyl; $R_c$ is selected from hydrogen or $OR_d$; and Y is selected from O, S, SO or $SO_2$.

Where possible, appropriate pharmaceutically acceptable salts of Formula I are included e.g.,

carboxylic or mineral acid addition salts where Formula I is basic and metal salts e.g. Na, K, $NH_4$ where Formula I is acidic.

The term alkyl, unless indicated otherwise, includes straight chain, branched chain and cycloalkyl groups. The term halogen or halo, unless otherwise indicated, includes Cl, Br, I and F.

A group of preferred compositions contain a compound of the Formula:

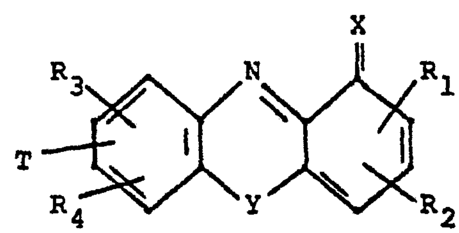

I(a)

More preferred Formula I(a) compounds are those wherein X is O or NH and Y is S, O, SO or $SO_2$. Still more preferred I(a) compounds are those having the Formula:

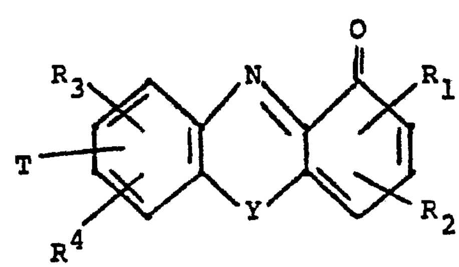

I(a)

wherein:

a) T, $R_3$ and $R_2$ are hydrogens,

b) T, $R_1$, $R_2$ are hydrogens,

c) T, $R_3$ and $R_4$ are hydrogens,

d) T, $R_1$, $R_2$ $R_3$ are hydrogens

or

e) T, $R_3$ $R_4$, $R_2$ are hydrogens.

wherein $R_1$, $R_2$, $R_3$, $R_4$ and T are as defined for Formula I.

Another group of preferred compositions contain compounds of the Formula I(b):

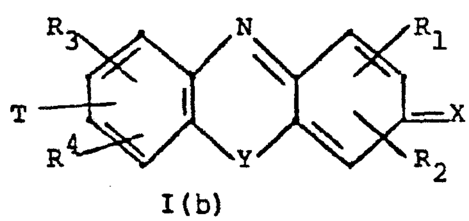

I(b)

More preferred compounds of Formula Ib are those having the Formula wherein X is O or NH and Y is O, S, SO, $SO_2$. Still more preferred compounds of Formula I(b) are those of the Formula I(c):

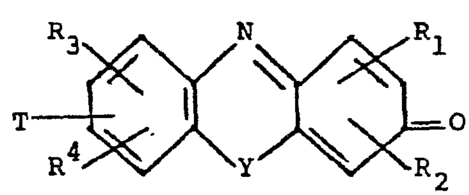

I(c)

wherein:

a) T, $R_3$ and $R_2$ are hydrogens,

b) T, $R_1$, $R_2$ are hydrogens,

c) T, $R_3$ and $R_4$ are hydrogens,

d) T, $R_1$, $R_2$ $R_3$ are hydrogens,

e) T, $R_3$ $R_4$, $R_2$ are hydrogens,

f) T, $R_3$ and $R_4$ are hydrogens and $R_1$ is in position 4,

g) T, $R_3$, $R_2$ are hydrogens and $R_1$ is in position 4,

h) T, $R_4$, $R_3$ $R_2$ are hydrogens and $R_1$ is in position 4,

i) $R_2$ and $R_3$ are hydrogens,

j) $R_1$ and $R_2$ are hydrogens,

k) $R_3$ and $R_4$ are hydrogens,

l) $R_2$ $R_3$ are hydrogens and T is in position 4,

m) T and $R_3$ are hydrogens and $R_2$ is in position 4,

n) T and $R_3$ are hydrogens and $R_1$ is in position 4 and $R_2$ is in position 2,

o) T and $R_3$ are hydrogens and $R_4$ is in position 7, $R_2$ is in position 4 and $R_1$ is in position 2.

(p) T is hydrogen, $R_1$, $R_2$, $R_3$, and $R_4$ are in positions 1, 2, 4 and 7, respectfully.

A particularly preierred series of Formula I(c) compounds are those in which n = 0 or 1 in the unit-$(CH_2)_nM$.

Examples of Formula I compounds useful in the present compositions are tabulated below. In each of the tables the numbers preceding the T and the $R_1$-$R_4$ definitions indicate the substituent position in the structure.

TABLE 1

Compounds of the Formula

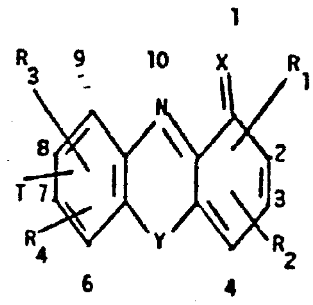

| Number | Y | X | $R_1$ | $R_2$ | $R_3$ | $R_4$ | T |
|---|---|---|---|---|---|---|---|
| 1 | O | O | 2-t-Bu | 8-t-Bu | 4-t-Bu | 6-t-Bu | H |
| 2 | O | O | 2-t-Bu | H | 4-Me | H | H |
| 3 | $N-CH_3$,S,O, Se,SO or $SO_2$ | O | 2-Cl | H | H | H | H |
| 4 | " | O | $2-SCF_3$ | H | H | H | H |
| 5 | " | O | 2-S 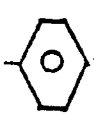 $CO_2H$ | H | H | H | H |
| 6 | " | O | 2-CN | H | H | H | H |
| 7 | " | O | H | $3-CO_2Et$ | H | H | H |
| 8 | " | O | H | 3-Cl | H | H | H |
| 9 | " | O | H | H | 4-Cl | H | H |
| 10 | " | O | H | H | $4-SO_2CH_3$ | H | H |
| 11 | " | O | 2-Cl | H | 4-Cl | H | H |
| 12 | " | NH | 2-Cl | H | 4-Cl | H | H |
| 13 | " | NH | H | H | H | H | H |
| 14 | N-CN | O | 2-Cl | H | 4-Cl | H | H |

TABLE 1 CONT'D

| Number | Y | X | $R_1$ | $R_2$ | $R_3$ | $R_4$ | T |
|---|---|---|---|---|---|---|---|
| 15 | S | O | H | H | H | H | H |
| 16 | S | O | 2Cl | 3-Cl | 4-Cl | 7-Cl | 9-Cl |
| 17 | S | O | 2-Br | 3-Br | 4-Br | 7-Br | 9-Br |
| 18 | S | O | H | H | H | 7-$SO_2CH_3$ | H |
| 19 | S | O | 2-Cl | H | 4-$SO_2CH_3$ | H | H |
| 20 | S | O | 2-F | H | 4-Cl | H | H |
| 21 | S | O | 2-Br | H | H | H | H |
| 22 | S | O | 2-$CF_3$ | H | H | H | H |
| 23 | S | O | 2-$SCF_3$ | H | H | H | H |
| 24 | S | O | 2-$SO_2CF_3$ | H | H | H | H |
| 25 | S | O | H | 3-Cl | H | H | H |
| 26 | S | O | H | 3-$CO_2Et$ | H | H | H |
| 27 | S | O | H | 3-$CO_2H$ | H | H | H |
| 28 | S | O | H | 3-CN | H | H | H |
| 29 | S | O | H | 3-$SCF_3$ | H | H | H |
| 30 | S | O | H | H | 4-Cl | H | H |
| 31 | S | O | H | H | 4-$SCF_3$ | H | H |
| 32 | S | O | H | H | 4-Cl | H | H |
| 33 | S | O | 2-Br | H | 4-Br | H | H |
| 34 | S | O | 2-Cl | H | H | 8-CN | H |
| 35 | S | O | 2-Cl | H | H | 8-$CO_2Et$ | H |
| 36 | S | O | 2-Cl | H | H | 8-$CO_2H$ | H |
| 37 | S | O | 2-Cl | H | H | 8-$CF_3$ | H |
| 38 | S | O | 2-Cl | H | H | 7-$SO_2CH_3$ | H |
| 39 | S | O | H | 3-$CONMe_2$ | H | H | H |
| 40 | S | O | 2-Cl | H | H | 7-$OCH_3$ | H |

TABLE 1 CONT'D

| Number | Y | X | $R_1$ | $R_2$ | $R_3$ | $R_4$ | T |
|---|---|---|---|---|---|---|---|
| 41 | S | O | 2-S- 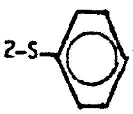 $CO_2H$ | H | H | H | H |
| 42 | S | O | 2-$SO_2CH_3$ | H | H | H | H |
| 43 | S | O | 2-$CH_2CH{=}CH_2$ | H | 4-$CH_2CH{=}CH_2$ | H | H |
| 44 | S | O | H | 3-$N(CH_3)_2$ | H | H | H |
| 45 | S | O | H | H | 4-Cl | 7-S-$C_6H_6$ | H |
| 46 | S | O | 2-$CH_2CO_2H$ | H | H | H | H |
| 47 | S | O | 2-Cl | H | 4-$SCH_2CO_2H$ | H | H |
| 48 | S | O | 2-$COCH_3$ | H | H | 7-$OCH_3$ | H |
| 49 | S | O | H | H | 4-CO-$C_6H_6$ | 7-$OCH_3$ | H |
| 50 | S | NH | 2-Cl | H | 4-Cl | H | H |
| 51 | S | NH | H | 3-$N(CH_3)_2$ | H | H | H |
| 52 | S | NH | 2-$SCH_3$ | H | 4-$SCH_3$ | H | H |
| 53 | S | O | H | H | H | H | H |
| 54 | S | NH | H | H | H | H | H |
| 55 | S | NH.HCl | H | H | H | H | H |
| 56 | S | O | H | H | H | H | H |
| 57 | O | O | H | H | H | H | H |
| 58 | O | NH | H | H | H | H | H |
| 59 | O | S | H | H | H | H | H |
| 60 | O | NH.HCl | H | H | H | H | H |
| 61 | Se | O | H | H | H | H | H |
| 62 | Se | NH | H | H | H | H | H |
| 63 | S[illegible] | S | H | H | H | H | H |
| 64 | [illegible] | NH.HCl | H | H | H | H | H |
| 65 | [illegible] | S | H | H | H | H | H |
| 66 | O | O | 4-Cl | H | H | H | H |

TABLE 1 CONT'D

| Number | Y | X | $R_1$ | $R_2$ | $R_3$ | $R_4$ | T |
|---|---|---|---|---|---|---|---|
| 67 | O | O | 4-Cl | H | 7-OMe | H | H |
| 68 | O | O | 4-Me | H | H | H | H |
| 69 | O | O | H | 2-Cl | H | H | H |
| 70 | O | O | 4-Cl | 2-S-pPAA* | H | H | H |
| 71 | Se | O | 4-Cl | H | H | H | H |
| 72 | Se | O | 4-Cl | H | 7-OMe | H | H |
| 73 | Se | O | 4-Me | H | H | H | H |
| 74 | Se | O | 4-Cl | 2-S-pPAA* | H | H | H |
| 75 | $N-CH_3$ | O | 4-Cl | H | H | H | H |
| 76 | $N-C_6H_6$ | O | 4-Cl | H | 7-OMe | H | H |
| 77 | N-H | O | 4-Cl | 2-S-pPAA* | H | H | H |
| 78 | S | O | 4-Cl | H | H | H | H |
| 79 | SO | O | H | H | H | H | H |
| 80 | $SO_2$ | O | H | H | H | H | H |
| 81 | $SO_2$ | O | 4-Cl | H | H | H | H |
| 82 | N-Me | O | H | H | H | H | H |
| 83 | N-Me | O | 4-Cl | H | H | H | H |
| 84 | N-Me | O | 4-Cl | H | 7-OMe | H | H |
| 85 | N-Me | O | 4-Br | H | 7-OMe | 2-OMe | H |
| 86 | NCN | O | 4-Br | H | 7-OMe | 2-OMe | H |
| 87 | NCN | O | 4-Cl | H | H | H | H |
| 88 | NH | O | 2-Cl | H | H | H | H |
| 89 | NH | O | 4-Cl | H | H | H | H |
| 90 | S | O | 2-t-Bu | 4-t-Bu | H | H | H |
| 91 | S | O | 2-t-Bu | 4-t-Bu | 9-OMe | H | H |
| 92 | S | O | 2-t-Bu | 4-t-Bu | 7-F | H | H |
| 93 | S | O | 2-t-Bu | 4-t-Bu | 7-Me | H | H |
| 94 | S | O | 2-t-Bu | 4-t-Bu | 7-SMe | H | H |

*p-PAA = para-phenylacetic acid

## TABLE 2

Compounds of the Formula

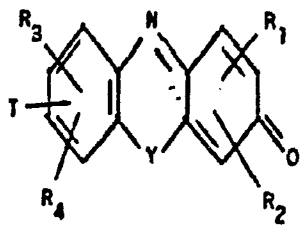

| Number | Y | $R_1$ | $R_2$ | $R_3$ | $R_4$ | T |
|---|---|---|---|---|---|---|
| 95 | S | H | H | H | H | H |
| 96 | S | 2-Cl | H | H | H | H |
| 97 | S | H | H | 6-Cl | H | H |
| 98 | S | H | H | 7-Cl | H | H |
| 99 | S | H | H | 8-Cl | H | H |
| 100 | S | H | H | 9-Cl | H | H |
| 101 | S | 1-Cl | H | H | H | H |
| 102 | S | 1-Cl | 4-Cl | H | H | H |
| 103 | S | 2-Cl | 4-Cl | H | H | 1-Cl |
| 104 | S | 2-N(Me)$_2$ | H | H | H | H |
| 105 | S | 2-SMe | H | H | H | H |
| 106 | S | 2-S-pPAA | H | H | H | H |
| 107 | S | 2-C(O)$CH_3$ | H | H | H | H |
| 108 | S | 2-OMe | H | H | H | H |
| 109 | S | H | H | H | 7-$CH_2CO_2H$ | H |
| 110 | S | H | H | H | 8-$CH_2COOH$ | H |
| 111 | S | H | 2-$SO_3$ | H | H | H |
| 112 | S | 2-N(Me)$_2$ | H | H | H | H |
| 113 | S | 2-SMe | H | H | H | H |
| 114 | S | 2-C(O)$CH_3$ | H | H | H | H |

TABLE 2 CONT'D

| Number | Y | $R_1$ | $R_2$ | $R_3$ | $R_4$ | T |
|---|---|---|---|---|---|---|
| 115 | S | 2-OMe | H | H | H | H |
| 116 | S | $2-CH_2CO_2H$ | H | H | H | H |
| 117 | S | $2-CH(CH_3)CO_2H$ | H | H | H | H |
| 118 | S | $4-CH_2COOH$ | H | H | H | H |
| 119 | S | $4-CH(CH_3)CO_2H$ | H | H | H | H |
| 120 | S | H | H | 7-OH | 6-propyl | H |
| 121 | S | 4-Cl | H | H | H | H |
| 122 | S | 4-F | H | H | H | H |
| 123 | S | 4-F | H | 7-Cl | H | H |
| 124 | S | 4-Et | H | H | H | H |
| 125 | S | 4-Et | H | 7-OMe | H | H |
| 126 | S | 4-Et | H | 7-Cl | H | H |
| 127 | S | 4-Cl | H | 7-OMe | H | H |
| 128 | S | 4-OMe | H | 7-Cl | H | H |
| 129 | S | 4-Cl | H | 6-Cl | H | H |
| 130 | S | 4-Cl | H | 8-Cl | H | H |
| 131 | S | 4-Cl | H | 9-Cl | H | H |
| 132 | S | 4-Cl | H | 6-OMe | H | H |
| 133 | S | 4-Cl | H | 8-OMe | H | H |
| 134 | S | 4-Cl | H | 9-Et | H | H |
| 135 | S | 4-Cl | H | 6-Et | H | H |
| 136 | S | 4-Cl | H | 7-Et | H | H |
| 137 | S | 4-Cl | H | 8-Et | H | H |
| 138 | S | 4-Cl | 1-Et | H | H | H |
| 139 | S | 4-Cl | 2-Et | H | H | H |
| 140 | S | 4-Cl | $1-CH_2COOH$ | H | H | H |
| 141 | S | 4-Cl | $2-CH_2COOH$ | H | H | H |
| 142 | S | 4-Cl | H | $6-CH_2COOH$ | H | H |

TABLE 2 CONT'D

| Number | Y | $R_1$ | $R_2$ | $R_3$ | $R_4$ | T |
|---|---|---|---|---|---|---|
| 143 | S | 4-Cl | H | 7-$CH_2COOH$ | H | H |
| 144 | S | 4-Cl | H | 8-$CH_2COOH$ | H | H |
| 145 | S | 4-Cl | 2-$N(Me)_2$ | H | H | H |
| 146 | S | 4-Cl | 1-$N(Me)_2$ | H | H | H |
| 147 | S | 4-Cl | 2-$N(Me)_2$ | 7-OMe | H | H |
| 148 | S | 4-Cl | 2-$N(Me)_2$ | 7-Cl | H | H |
| 149 | S | 4-Cl | 2-SMe | H | H | H |
| 150 | S | 4-Cl | 2-$SCH_2COOH$ | H | H | H |
| 151 | S | 4-Cl | 2-S-pPAA | H | H | H |
| 152 | S | 4-Cl | 1-S-pPAA | H | H | H |
| 153 | S | 4-Cl | 2-S-pPAA | 7-OMe | H | H |
| 154 | S | 4-Cl | 2-$SO_3H$ | H | H | H |
| 155 | S | 4-Cl | 2-OMe | H | H | H |
| 156 | S | 4-Cl | 2-OMe | 7-Cl | H | H |
| 157 | S | 4-Cl | H | 7F | H | H |
| 158 | S | 4-OMe | H | 7-OMe | H | H |
| 159 | S | 4-OMe | H | 7-Me | H | H |
| 160 | S | 4-OMe | 2-SMe | H | H | H |
| 161 | S | 4-SMe | H | H | H | H |
| 162 | S | 4-Br | H | H | H | H |
| 163 | S | 4-I | H | H | H | H |
| 164 | S | 4-Br | H | 7-OMe | H | H |
| 165 | S | 4-I | H | 7-OMe | H | H |
| 166 | S | 4-Br | 2-Me | H | H | H |
| 167 | S | 4-I | 2-Me | H | H | H |
| 168 | S | 4-Cl | H | 7/8-$(CH_2)_4$- | | H |
| 169 | S | 4-Cl | H | 7/8-$(CH_2)_3$- | | H |
| 170 | S | 4-Br | 2-OMe | 7-OMe | H | H |

TABLE 2 CONT'D

| Number | Y | $R_1$ | $R_2$ | $R_3$ | $R_4$ | T |
|---|---|---|---|---|---|---|
| 171 | S | 2-OMe | 7-OMe | H | H | H |
| 172 | S | 1-OMe | 7-Ome | H | H | H |
| 173 | S | 2-OMe | 7-OMe | H | H | 1-Br |
| 174 | S | 1-OMe | 7-OMe | H | H | 2-Br |
| 175 | S | 1-OMe | 7-OMe | H | H | 4-Br |
| 176 | S | 1-OMe | 7-OMe | H | H | 2-Cl |
| 177 | S | 1-OMe | 7-OMe | H | H | 4-Cl |
| 178 | S | 2-OMe | 7-OMe | H | H | 1-Cl |
| 179 | S | 2-OMe | 7-OMe | H | H | 4-Cl |
| 180 | S | 2-OEt | 7-OEt | H | H | 1-Br |
| 181 | S | 2-OEt | 7-OEt | H | H | 4-Br |
| 182 | S | 2-OEt | 7-OEt | H | H | 1-Cl |
| 183 | S | 2-OEt | 7-OEt | H | H | 4-Cl |
| 184 | S | 2-OMe | 7-OMe | 8-OMe | H | 1-Br |
| 185 | S | 2-OMe | 7-OMe | 8-OMe | H | 4-Br |
| 186 | S | 2-OMe | 7-OMe | H | H | 4-F |
| 187 | S | 2-OMe | 7-OMe | H | H | 4-$CF_3$ |
| 188 | S | 2-OMe | 7-OEt | H | H | 4-Br |
| 189 | S | 2-OMe | 7-OEt | H | H | 4-Cl |
| 190 | S | 2-OMe | 7-OEt | H | H | 4-F |
| 191 | S | 2-OMe | 7-OEt | H | H | 4-$CF_3$ |
| 192 | S | 2-OEt | 7-OMe | H | H | 4-Br |
| 193 | S | 2-OEt | 7-Ome | H | H | 4-Cl |
| 194 | S | 2-OEt | 7-OMe | H | H | 4-F |
| 195 | S | 2-OEt | 7-OMe | H | H | 4-$CF_3$ |
| 196 | S | 1-OMe | 2-OMe | 7-OMe | H | 4-Br |
| 197 | S | 1-OMe | 2-OMe | H | H | |
| 198 | S | 1-OMe | 2-OMe | H | H | 1-Br |
| 199 | O | | Same as Numbers 90-198 | | | |

TABLE 2 CONT'D

| Number | Y | $R_1$ | $R_2$ | $R_3$ | $R_4$ | T |
|---|---|---|---|---|---|---|
| 200 | $SO_2$ | 4-OH | H | H | H | H |
| 201 | $SO_2$ | 1-OMe | 2-OMe | $4-CH_3$ | H | H |
| 202 | $SO_2$ | 2-OMe | 7-OMe | 4-OH | H | H |
| 203 | $SO_2$ | 2-OMe | 4-OH | H | H | H |
| 204 | $SO_2$ | 1-OMe | 4-OH | H | H | H |
| 205 | $SO_2$ | 2-Me | 4-OH | H | H | H |
| 206 | $SO_2$ | 2-Cl | 4-OH | H | H | H |
| 207 | $SO_2$ | 2-OEt | 7-OEt | 4-OH | H | H |
| 208 | $SO_2$ | $2-SO_2Me$ | 4-OH | H | H | H |
| 209 | $SO_2$ | 4-OMe | H | H | H | H |
| 210 | $SO_2$ | 2-OMe | 4-OMe | 7-OMe | H | H |
| 211 | O | $1-CO_2H$ | 4-OH | $7-NMe_2$ | H | H |
| 212 | O | 1-Cl | 2-Cl | 4-Cl | H | 7-Cl |
| 213 | S | 9-OMe | H | H | H | H |
| 214 | S | 2-OMe | H | H | H | H |
| 215 | S | 2-OMe | 4-OMe | H | H | H |
| 216 | S | 1-OMe | 2-OMe | 4-Me | H | H |
| 217 | S | 4-OMe | H | H | H | H |
| 218 | S | 1-OMe | 7-OMe | H | H | 4-Br |
| 219 | S | 1-OMe | 7-OMe | 2-Cl | H | 4-Cl |
| 220 | S | 1-OMe | 7-OMe | H | H | 4-Cl |
| 221 | S | 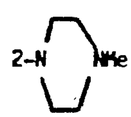 | 7-OMe | H | H | H |
| 222 | S | 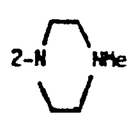 | 7-[illegible]Me | H | H | 4-Br |
| 223 | $SO_2$ | 2-OMe | 4-OH | 7-OMe | H | H |

TABLE 2 CONT'D

| Number | Y | $R_1$ | $R_2$ | $R_3$ | $R_4$ | T |
|---|---|---|---|---|---|---|
| 224 | $SO_2$ | 1-NHPr | 4-NHPr | H | H | H |
| | $SO_2$ | | | | | |
| 225 | $SO_2$ | 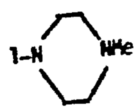 | 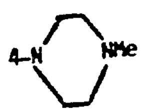 | ? | H | H |
| 226 | $SO_2$ | 2-OMe | 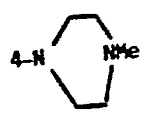 | 7-OMe | H | H |
| 227 | $SO_2$ | 2-OMe | 7-OMe | H | H | H |
| 228 | $SO_2$ | 2-OMe | 4-NHPr | 7-OMe | H | H |
| 229 | S | 1-NHPr | 4-NHPr | H | H | H |
| 230 | S | 1-NHPr | 4-NHPr | 7-OMe | H | H |
| 231 | S | 1-NHPr | 4-NHPr | H | H | H |
| 232 | S | 2-NHPr | 4-NHPr | 7-OMe | H | H |
| 233 | S | 2-OMe | $4-NH_2$ | 7-OMe | H | H |
| 234 | S | 2-OMe | 4-NHPr | 7-OMe | H | H |
| 235 | O | 1-OMe | 4-Cl | 7-OMe | H | H |
| 236 | O | 1-OMe | 4-Br | 7-OMe | H | H |
| 237 | O | 1-NHPr | 4-NHPr | H | H | H |
| 238 | $SO_2$ | 1-OMe | 4-CN | 7-OMe | H | H |
| 239 | $SO_2$ | 2-OMe | $4-NHCH_2CO_2R$* | 7-OMe | H | H |
| 240 | $SO_2$ | 2-OMe | 4-S-7-Bu | 7-OMe | H | H |
| 241 | $SO_2$ | 2-OMe | $4-CH_2CO_2R$* | 7-OMe | H | H |
| 242 | $SO_2$ | 2-OMe | $4-SO_2Me$ | 7-OMe | H | H |
| 243 | S | 2-S-n-Bu | H | H | H | H |
| 244 | S | 4-S-n-Bu | H | H | H | H |
| 245 | S | 2-Me | 4-S-n-Bu | H | H | H |

* R is H or $C_1C_4$ alkyl.

TABLE 2 CONT'D

| Number | Y | $R_1$ | $R_2$ | $R_3$ | $R_4$ | T |
|---|---|---|---|---|---|---|
| 246 | S | 2-OMe | 7-Me | H | H | 4-Br |
| 247 | S | 2-OMe | $7-CF_3$ | H | H | 4-Br |
| 248 | S | 2-OMe | 7-F | H | H | 4-Br |
| 249 | S | 2-OMe | 7-Cl | H | H | 4-Br |
| 250 | S | 2-OMe | 7-Br | H | H | 4-Br |
| 251 | S | 2-OMe | $7-NMe_2$ | H | H | 4-Br |
| 252 | S | 2-OMe | 7-SMe | H | H | 4-Br |
| 253 | S | 2-OMe | $7-SO_2Me$ | H | H | 4-Br |
| 254 | S | 2-OMe | 7-Ph | H | H | 4-Br |
| 255 | S | 1-Me | H | H | H | H |
| 256 | S | 2-Me | H | H | H | H |
| 257 | S | 2-OEt | H | H | H | H |
| 258 | S | 7-Cl | H | H | H | H |
| 259 | S | 9-Cl | H | H | H | H |
| 260 | S | 7-F | H | H | H | H |
| 261 | S | 7-Me | H | H | H | H |
| 262 | S | 7-OMe | H | H | H | H |
| 263 | S | 2-Cl | H | H | H | H |
| 264 | S | 1-Me | 7-Me | H | H | H |
| 265 | S | 1-Me | 7-Me | H | H | H |
| 266 | S | 2-OMe | 7-OEt | H | H | H |
| 267 | O | $2-NH_2$ | H | H | H | H |
| 268 | O | 7-OH | H | H | H | H |
| 269 | O | COMe | H | H | H | H |
| 270 | $SO_2$ | 2-OMe | 7-OMe | H | H | 4-Br |
| 271 | S | 2-NHPr | 4-NHPr | H | H | H |
| 272 | S | 2-Cl | H | H | H | 4-Cl |
| 273 | S | 1-Me | 7-Me | H | H | 4-Cl |

TABLE 3

Compounds of the Formula

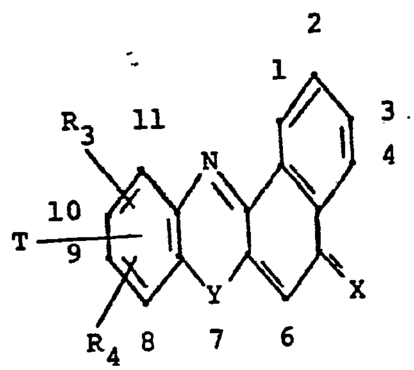

| ..mple | Y | X | $R_3$ | $R_4$ | T |
|---|---|---|---|---|---|
| 1 | O | O | H | H | H |
| 2 | S | O | H | H | H |
| 3 | SO | O | H | H | H |
| 4 | $SO_2$ | O | H | H | H |
| 5 | SO | O | H | H | 6-Cl |
| 6 | S | O | 6-$COCH_3$ | H | H |
| 7 | S | O | 6-$CH_3$ | H | H |
| 8 | $SO_2$ | O | 6-OH | H | H |
| 9 | $SO_2$ | O | 6-OMe | H | H |
| 10 | S | O | 9-OMe | H | H |
| 11 | S | O | 6-OH | H | H |
| 12 | S | O | 6-OMe | H | H |
| 13 | S | O | 6-NHCOMe | H | H |
| 14 | S | O | 6-NHPh | H | H |
| 15 | S | O | H | H | 6-Br |
| 16 | S | O | 6-NHMe | H | H |
| 17 | S | O | 6-NH-t-Bu | H | H |
| 18 | S | O | 6-NH-COMe | H | 9-Cl |
| 19 | S | O | 6-NH-COMe | 9-OMe | H |

TABLE 3 CONT'D

| Example | Y | X | $R_3$ | $R_4$ | T |
|---|---|---|---|---|---|
| 20 | S | O | 6-NHPh-p-Br | H | 9-Cl |
| 21 | O | O | H | H | 6-Cl |
| 22 | O | O | H | H | 6-Br |
| 23 | O | O | 9-OMe | H | 6-Br |
| 24 | O | O | 9-OMe | 6-NHPr | H |
| 25 | S | O | 6-$CF_3$ | H | H |
| 26 | S | O | 6-S-n-Bu | H | H |
| 27 | S | O | 6-OMe | H | 9-Cl |
| 28 | S | O | 9-OMe | H | 6-Cl |
| 29 | S | O | 6-OMe | 9-OMe | H |
| 30 | S | O | 6-Cl | 9-Me | 11-Br |
| 31 | S | O | 6-NHPh | 9-Me | 11-Br |
| 32 | S | O | 6-Me | H | H |
| 33 | O | NH | 9-$NMe_2$ | 10-Me | H |
| 34 | O | NH | 9-$N(Et)_2$ | H | H |

In addition to the specific compounds described above encompassed by Formula I, these further compounds of Formula I are also within the scope of the invention:

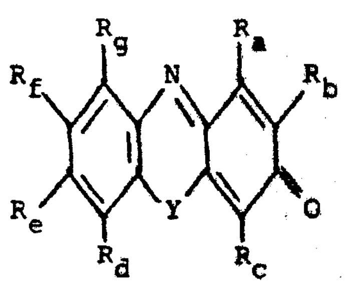

Tables 4, 5 and 6 further describe these compounds:

TABLE 4

FORMULA I COMPOUND

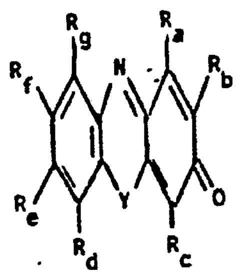

| Y | $R_a$ | $R_b$ | $R_c$ | $R_d$ | $R_e$ | $R_f$ | $R_g$ |
|---|---|---|---|---|---|---|---|
| S | H | H | $CF_3$ | H | H | H | H |
| S | H | $CF_3$ | $CF_3$ | H | H | H | H |
| S | $CF_3$ | H | H | H | H | H | H |
| S | H | F | H | H | H | H | H |
| S | H | $SCH_3$ | H | H | H | H | H |
| S | H | OH | H | H | H | H | H |
| S | H | H | F | H | H | H | H |
| S | H | H | $OCH_3$ | H | H | H | H |
| S | H | H | $SCF_3$ | H | H | H | H |
| S | H | H | CN | H | H | H | H |
| S | H | H | CHO | H | H | H | H |
| S | H | H | $COCF_3$ | H | H | H | H |
| S | H | H | H | H | $SCH_3$ | H | H |
| S | H | H | H | H | $OCH_3$ | H | H |
| S | H | H | H | H | $CO_2CH_3$ | H | H |
| S | H | H | H | H | $CO_2H$ | H | H |
| S | H | H | H | H | CN | H | H |
| S | H | H | H | H | CHO | H | H |
| S | H | H | H | H | $CONH_2$ | H | H |

TABLE 4 CONT'D

| Y | $R_a$ | $R_b$ | $R_c$ | $R_d$ | $R_e$ | $R_f$ | $R_g$ |
|---|---|---|---|---|---|---|---|
| S | H | H | H | H | $CH_2OH$ | H | H |
| S | H | H | H | H | $CF_3$ | H | H |
| S | $CH_3$ | H | Cl | H | H | H | H |
| S | H | $CH_3$ | Cl | H | H | H | H |
| S | H | H | Cl | H | F | H | H |
| S | H | Cl | Cl | H | F | H | H |
| S | H | $CH_3$ | H | H | F | H | H |
| S | $CH_3$ | H | H | H | F | H | H |
| S | H | H | Cl | H | OMe | H | H |
| S | H | H | Cl | H | $CF_3$ | H | H |
| S | H | H | Cl | H | $CO_2Me$ | H | H |
| S | H | H | Cl | H | $CO_2H$ | H | H |
| S | H | H | Cl | H | CN | H | H |
| S | H | H | Cl | H | CHO | H | H |
| S | H | H | Cl | H | $CONH_2$ | H | H |
| S | H | H | Cl | H | $CH_2OH$ | H | H |
| S | H | H | $OCH_3$ | H | Cl | H | H |
| S | H | H | $CF_3$ | H | Cl | H | H |
| S | H | OEt | Cl | H | H | H | H |
| S | H | OiPr | H | H | H | H | H |
| S | OMe | H | Cl | H | H | H | H |
| S | OEt | H | Cl | H | H | H | H |
| S | H | OiPr | Cl | H | H | H | H |
| S | H | O-benzyl | Cl | H | H | H | H |
| S | H | $OCH_3$ | Cl | H | H | H | H |
| S | H | OEt | H | H | F | H | H |
| S | H | OEt | H | H | $CH_3$ | H | H |

TABLE 4 CONT'D

| Y | $R_a$ | $R_b$ | $R_c$ | $R_d$ | $R_e$ | $R_f$ | $R_g$ |
|---|---|---|---|---|---|---|---|
| S | H | OEt | Cl | H | F | H | H |
| S | H | OEt | Cl | H | $CH_3$ | H | H |
| S | H | H | Cl | H | $CH_3$ | H | H |
| S | H | $CH_3$ | I | H | H | H | H |
| S | H | $CH_3$ | Br | H | H | H | H |
| S | $CH_3$ | H | H | H | $CH_3$ | H | H |
| S | H | $CH_3$ | H | H | $CH_3$ | H | H |
| S | $CH_3$ | H | Cl | H | $CH_3$ | H | H |
| S | H | $CH_3$ | Cl | H | $CH_3$ | H | H |
| S | Cl | H | Cl | H | F | H | H |
| | H | OMe | Br | H | OMe | H | H |
| , | H | OMe | Cl | H | OMe | H | H |
| S | H | OEt | Br | H | OEt | H | H |
| S | H | OEt | Cl | H | OEt | H | H |
| S | H | OMe | Cl | H | OEt | H | H |
| S | H | OMe | H | H | SMe | H | H |
| O | H | OMe | Br | H | OMe | H | H |
| O | H | OMe | Cl | H | OMe | H | H |
| O | H | H | Cl | H | H | H | H |
| $SO_2$ | H | H | OH | H | H | H | H |
| $SO_2$ | H | OMe | OH | H | OMe | H | H |
| $SO_2$ | OMe | OMe | Me | H | H | H | H |
| $SO_2$ | H | H | OMe | H | H | H | H |
| $SO_2$ | H | OMe | OMe | H | OMe | H | H |
| S | H | H | H | H | H | H | $OCH_3$ |
| S | H | $OCH_3$ | H | H | F | H | H |
| S | H | $OCH_3$ | $OCH_3$ | H | H | H | H |

TABLE 4 CONT'D

| Y | $R_a$ | $R_b$ | $R_c$ | $R_d$ | $R_e$ | $R_f$ | $R_g$ |
|---|---|---|---|---|---|---|---|
| S | $OCH_3$ | $OCH_3$ | Me | H | H | H | H |
| S | H | H | $COCH_3$ | H | H | H | H |
| S | $OCH_3$ | H | Br | H | $OCH_3$ | H | H |
| S | $OCH_3$ | Cl | Cl | H | $OCH_3$ | H | H |
| S | $OCH_3$ | H | Cl | H | $OCH_3$H | H | H |
| S | H | 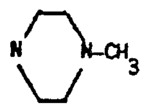 | H | H | $OCH_3$ | H | H |
| S | H | 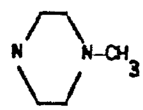 | Br | H | $OCH_3$ | H | H |
| $SO_2$ | H | $OCH_3$ | OH | H | $OCH_3$ | H | H |
| $SO_2$ | NHPr | H | NHPr | H | H | H | H |
| $SO_2$ | 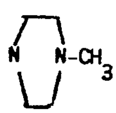 | H | 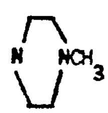 | H | H | H | H |
| $SO_2$ | H | $OCH_3$ | 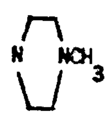 | H | $OCH_3$ | H | H |
| $SO_2$ | H | $OCH_3$ | Br | H | $OCH_3$ | H | H |
| S | NHPr | H | NHPr | H | H | H | H |
| S | NHPr | H | NHPr | H | $OCH_3$ | H | H |

TABLE 4 CONT'D

| Y | $R_a$ | $R_b$ | $R_c$ | $R_d$ | $R_e$ | $R_f$ | $R_g$ |
|---|---|---|---|---|---|---|---|
| S | H | NHPr | NHPr | H | H | H | H |
| S | H | NHPr | NHPr | H | $OCH_3$ | H | H |
| S | H | $OCH_3$ | $NH_2$ | H | $OCH_3$ | H | H |
| S | H | $OCH_3$ | NHPr | H | $OCH_3$ | H | H |
| $SO_2$ | H | $OCH_3$ | NHPr | H | $OCH_3$ | H | H |
| O | OCH | H | Cl | H | $OCH_3$ | H | H |
| O | OCH | H | Br | H | $OCH_3$ | H | H |
| O | NHP | H | NHPr | H | H | H | H |
| $SO_2$ | H | $OCH_3$ | CN | H | $OCH_3$ | H | H |
| $SO_2$ | H | $OCH_3$ | $NHCH_2CO_2R$* | H | $OCH_3$ | H | H |
| $SO_2$ | H | $OCH_3$ | S-n-Bu | H | $OCH_3$ | H | H |
| $SO_2$ | H | $OCH_3$ | $CH_2CO_2R$* | H | $OCH_3$ | H | H |
| $SO_2$ | H | $OCH_3$ | $SO_2CH_3$ | H | $OCH_3$ | H | H |
| S | H | S-n-Bu | H | H | H | H | H |
| S | H | H | S-n-Bu | H | H | H | H |
| S | H | $CH_3$ | S-n-Bu | H | H | H | H |
| S | H | OMe | Br | H | $CF_3$ | H | H |
| S | H | OMe | Br | H | F | H | H |
| S | [illegible] | OMe | Br | H | Cl | H | H |
| S | [illegible] | OMe | Br | H | Br | H | H |
| S | [illegible] | OMe | Br | H | $NMe_2$ | H | H |
| S | H | OMe | Br | H | SMe | H | H |
| S | H | OMe | Br | H | $SO_2Me$ | H | H |
| S | H | OMe | Br | H | Ph | H | H |
| S | H | H | H | Cl | OMe | H | H |
| S | H | OMe | Br | H | Me | H | H |

;

* R is H or $C_1$-$C_4$ alkyl.

Table 5 describes the compounds useful in the present invention having four rings.

TABLE 5

COMPOUNDS OF FORMULA II

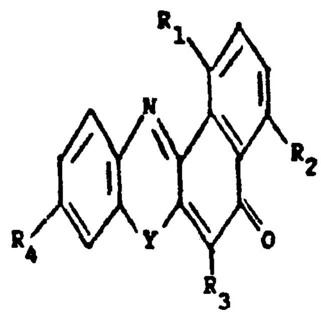

II

| Y | $R_1$ | $R_2$ | $R_3$ | $R_4$ |
|---|---|---|---|---|
| S | H | H | S-n-$CH_4H_9$ | H |
| S | OH | H | $CH_3$ | H |
| S | $OCH_3$ | H | $CH_3$ | H |
| S | H | H | F | H |
| S | H | H | $CF_3$ | H |
| S | H | H | Cl | $CF_3$ |
| S | H | H | Cl | $SCH_3$ |
| S | H | H | Br | Cl |
| S | H | H | $CH_3$ | Br |
| S | H | H | F | Br |
| S | H | H | $COCH_3$ | Cl |
| S | H | H | $CF_3$ | $CH_3$ |
| S | H | H | S-n-$C_4H_9$ | $CH_3$ |
| S | H | H | CF | Cl |
| S | H | H | Cl | *$CH_2COOR$ |

TABLE 5 CONT'D

| Y | $R_1$ | $R_2$ | $R_3$ | $R_4$ |
|---|---|---|---|---|
| S | H | H | Cl | *CH(Me)$CO_2$R |
| S | H | H | Cl | $COCH_3$ |
| S | H | H | H | Cl |
| S | H | H | H | Br |
| S | H | H | H | F |
| S | H | H | H | $CF_3$ |
| S | H | H | H | $CH_3$ |
| S | H | H | H | $CH_2OH$ |
| S | H | H | H | $OCH_3$ |
| S | H | H | H | $SCH_3$ |
| S | H | H | H | *COOR |
| S | H | H | H | *$CH_2CO_2$R |
| S | H | H | H | *CH(Me)$CO_2$R |
| $SO_2$ | H | H | NHPr | H |
| $SO_2$ | H | H | 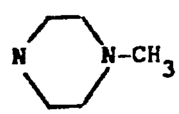 | H |
| $SO_2$ | H | H | $NH_2$ | H |
| $SO_2$ | H | H | NHPr | $OCH_3$ |
| S | -1,4-dihydro | | H | |
| S | H | H | NHPr | $OCH_3$ |
| O | H | H | Cl | H |
| O | H | H | Br | H |
| O | H | H | Br | $OCH_3$ |
| O | H | H | NHPr | $OCH_3$ |

*R is H or $C_{1-4}$alkyl

Table 6 describes additional compounds useful in the present invention.

TABLE 6

COMPOUNDS OF FORMULA I

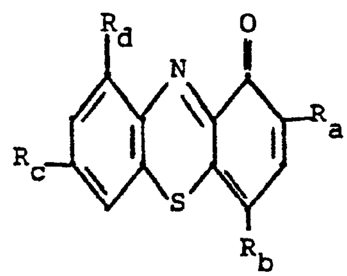

I

| $R_a$ | $R_b$ | $R_c$ | $R_d$ |
|---|---|---|---|
| t-Bu | t-Bu | H | H |
| t-Bu | t-Bu | F | H |
| t-Bu | t-Bu | Me | H |
| t-Bu | t-Bu | SMe | H |
| t-Bu | t-Bu | H | OMe |

Formula I includes both novel and known compounds. These compounds may be prepared by any process available to the skilled artisan.

One such process for compounds where X = 0 involves the oxidation of the appropriate phenothiazine as illustrated by the following equations.

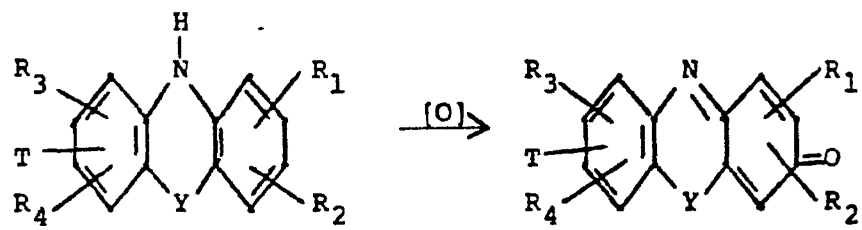


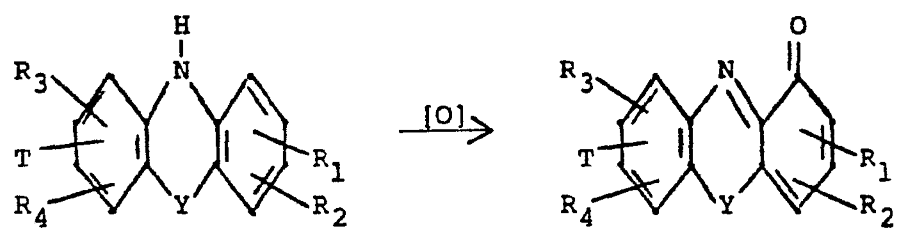


Various oxidizing agents and systems are taught in the art, e.g. $PbO_2$, $HNO_3$, $K_2Cr_2O_7$, $K_2Cr_2O_7$, iodine, $FeCl_3$ and the like.

Another process useful for preparing some Formula I compounds containing halogen substituents is by direct halogenation of an appropriate phenothiazone or analog thereof as illustrated by the following equation.

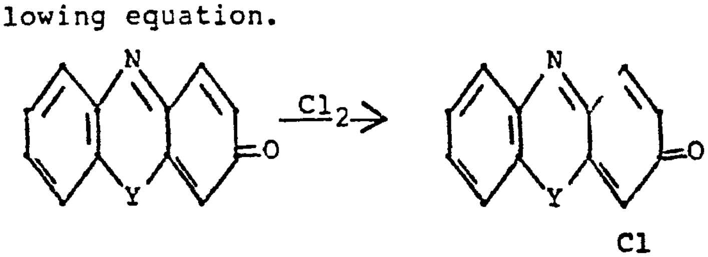

Still another process useful for preparing many of the Formula I compounds is by the reaction of an appropriate aniline with an appropriate quinone as illustrated by the following equation:

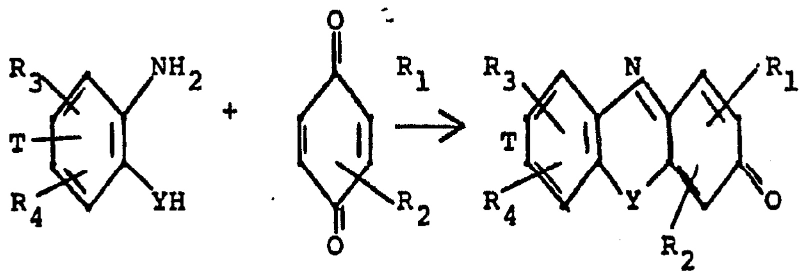

This general process is described in the literature.

A specific process for preparing the intermediate phenothiazin-3-one is illustrated by the following equation:

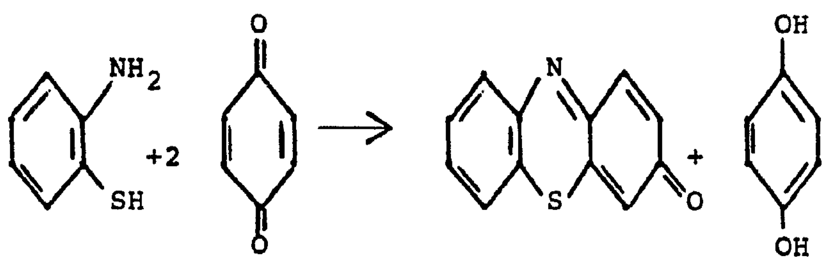

A B C D

The process requires the use of 2 moles of quinone per mole of aniline. Any suitable solvent may be used. Example of such solvents are acetic acid, lower alkanols, acetic acid/$H_2O$, loweralkanol/water, other polar solvents and the like. A preferred solvent is one which will dissolve A, B and D and in which C is substantially insoluble. The reaction is readily carried out at room temperature - lower temperatures, e.g. as low as -10°C, may be used - elevated temperatures may also be used but are not required.

This process is more fully described in our copending application U.S. Serial Number 459,923, filed on January 21, 1983, which is hereby incorporated by reference.

Another useful process to prepare certain of the compounds of the present invention is the oxidation of certain phenothiazines or benzo[a]-phenothiazines by standard oxidizing agents such as potassium dichromate, $NaClO_2$, 2,3-dichloro-5,6-dicyano-1,4-benzoquinone (DDQ), and the like. Several of these benzo[a]phenothiazines and phenothiazines are described in our copending applications U.S. Serial Numbers 539,342 and 539,215, respectively, both filed on October 5, 1983, which are hereby incorporated by reference.

Examples for the preparation and use of Formulae I and II compounds follow. These examples are provided merely as an aid to understand the instant invention and not as a limitation. All temperatures are in degrees Celsius and are uncorrected.

EXAMPLE 1

Method A: 3H-Phenothiazin-3-one

To a stirring suspension of 1.72 kg (16 mol) of p-benzoquinone in 13 liters MeOH at room temperature was added slowly a solution of 1.0 kg (8 mol) of 2-aminothiophenol in 600 ml MeOH over a period of 1 hour. The resulting red mixture was stirred at room temperature for another 2 hours and then the product 3H-phenothiaz-3-one was filtered off. This 3H-phenothiazin-3-one was washed thoroughly with methanol and dried to give 1.07 kg of 3H-phenothiazin-3-one (61.49% yield), m.p. 157-159°C.

Method B:

To a stirring solution of 1.1 kg of ceric ammonium nitrate in 12.5 liters of $H_2O$ and 1.25 liters of HOAc at 10°C was added dropwise a solution of 100 g of phenothiazine in 500 ml acetone over a period of 20 minutes. The resulting mixture was stirred for another 20 minutes and the product 3H-phenothiazin-3-one was then filtered off. The filtered 3H-phenothiazin-3-one was washed with water thoroughly and dried to give 92 g of crude 3H-phenothiazin-3-one. The crude 3H-phenothiazin-3-one was extracted with minimum volume of $CH_2Cl_2$. Upon dilution of the $CH_2Cl_2$ solution with 10 times the volume of cyclohexane, a precipitate was formed which was filtered and dried to afford 35 g of 3H-phenothiazin-3-one.

EXAMPLE 2

4-Chloro-3H-phenothiazin-3-one

To a stirring solution of 500 g (2.34 mol) of 3H-phenothiazin-3-one in 12.5 liters of glacial acetic acid was added 1.25 kg of potassium dichromate. The mixture was stirred at room temperature for 1/2 hour. To this resulting mixture was then added 2.34 mol of a 1M solution of chlorine in glacial acetic acid dropwise over a period of 4 hours. The progress of the reaction was monitored by tlc to ensure no excess chlorine was added. After addition of chlorine was completed the mixture was stirred at room temperature for another 1/2 hour and was then poured into 20 liters of $H_2O$ with vigorous stirring. The 4-chloro-3H-phenothiazin-3-one precipitated was allowed to settle overnight. The majority of the

aqueous solution was siphoned and discarded and the rest was filtered. The filtered precipitate was washed thoroughly with water and then rinsed with methanol and was allowed to dry to give 504 g crude 4-chloro-3H-phenothiazin-3-one which was recrystallized from toluene, m.p. 221°.

EXAMPLE 3

4-Chloro-2,7-dimethoxy-3H-phenothiazin-3-one

Following the procedure described in Example 2 but substituting 2,7-dimethoxy-3H-phenothiazin-3-one for 3H-phenothiazin-3-one, the title compound was obtained, m.p. 264°C.

Analysis, calculated:

C, 54.63; H, 3.27; N, 4.55; S, 10.42; Cl, 11.52.

Observed: C, 54.64; H, 3.31; N, 4.53; S, 10.60; Cl, 11.69.

EXAMPLE 4

4-Chloro-1,7-dimethyl-3H-phenothiazin-3-one

Following the procedure described in Example 2 but substituting 1,7-dimethyl-3H-phenothiazin-3-one for 3H-phenothiazi[illegible] for 3H-phenothiazin-3-one the title compound was obtained, m.p. 215-218°C.

Analysis, calculated:

C, 60.98, H, 3.66; N, 5.08; S, 11.63; Cl, 12.86.

Observed: C, 60.78; H, 3.75; N, 4.99; S, 11.79; Cl, 13.01.

EXAMPLE 5

4-Chloro-2,7-dimethyl-3H-phenothiazin-3-one

Following the procedure described in Example 2 but substituting 2,7-dimethyl-3H-phenothiazin-3-one for 3H-phenothiazin-3-one the title compound was obtained, m.p. 193-195°C.

Analysis, calculated:
C, 60.98; H, 3.66; N, 5.08; S, 11.63; Cl, 12.86.
Observed: C, 60.89; H, 3.79; N, 5.22; S, 11.63; Cl, 12.50.

EXAMPLE 6

4-Chloro-2-methyl-3H-phenothiazin-3-one

Following the procedure described in Example 2 but substituting 2-methyl-3H-phenothiazin-3-one for 3H-phenothiazin-3-one, the title compound was obtained, m.p. 206°C.

Analysis, calculated:
C, 59.66; H, 3.08; N, 5.35; S, 12.25; Cl, 13.55.
Observed: C, 59.59; H, 3.35; N, 5.32; S, 12.64; Cl, 13.27.

EXAMPLE 7

4-Chloro-7-methyl-3H-phenothiazin-3-one

Following the procedure described in Example 2 but substituting 7-methyl-3H-phenothiazin-3-one for 3H-phenothiazin-3-one, the title compound was obtained, m.p. 218°C.

Analysis, calculated:

C, 59.66; H, 3.08; N, 5.35; S, 12.25; Cl, 13.55.

Observed: C, 59.48; H, 3.17; N, 5.27; S, 12.40; Cl, 13.63.

## EXAMPLE 8

4-Chloro-7-ethoxy-2-methoxy-3H-phenothiazin-3-one

Following the procedure described in Example 2 but substituting 7-ethoxy-2-methoxy-3H-phenothiazin-3-one for 3H-phenothiazin-3-one, the title compound was obtained, m.p. 236-239°C.

Analysis calculated:

C, 55.99; H, 3.76; N, 4.35; S, 9.96; Cl, 11.02.

Observed: C, 56.05; H, 3.93; N, 4.37; S, 10.11; Cl, 10.99.

## EXAMPLE 9

4-Bromo-2-methyl-3H-phenothiazin-3-one

Following the procedure described in Example 2 but substituting 2-methyl-3H-phenothiazin-3-one for 3H-phenothiazin-3-one and substituting bromine for chlorine, the title compound was obtained, m.p. 190°C.

Analysis, calculated:

C, 50.99; H, 2.63; N, 4.57; S, 10.47; Br, 26.09.

Observed: C, 50.97; H, 2.69; N, 4.61; S, 10.56; Br, 26.24.

EXAMPLE 10

9-Methoxy-3H-phenothiazin-3-one

To a suspension of p-benzoquinone (3.0 g) in 15 ml methanol was added 2-amino-3-methoxythiophenol (2.2 g) dissolved in 10 ml methanol. The mixture was stirred at room temperature for 45 minutes and concentrated *in vacuo*. The residue was triturated with ether and filtered. The resulting dark solid was chromatographed on silica gel and eluted with EtOAc, to afford the desired compound, m.p. 206-207°.

Analysis, calculated:
C, 64.18; H, 3.73; N, 5.76; S, 13.18.
Observed: C, 64.10; H, 3.83; N, 5.69; S, 13.40.

EXAMPLE 11

7-Fluoro-3H-phenothiazin-3-one

Following the procedure described in Example 10 but substituting 2-amino-5-fluorothiophenol for 2-amino-3-methoxythiophenol, the title compound was obtained, m.p. 240°C.

Analysis, calculated:
C, 62.33; H, 2.61; N, 6.06; S, 13.86; F, 8.21.
Observed: C, 62.26; H, 2.70; N, 6.05; S, 14.04; F, 8.06.

EXAMPLE 12

4-Chloro-7-fluoro-3H-phenothiazin-3-one

Following the procedure described in Example 2 but substituting 7-fluoro-3H-phenothiazin-3-one for 3H-phenothiazin-3-one, the title compound was obtained, m.p. 250-255°C.

Analysis, calculated:

C, 54.25; H, 1.90; N, 5.27; S, 12.07; F, 7.15; Cl, 13.34.

Observed: C, 54.10; H, 2.01; N, 5.35; S, 12.20; F, 7.20; Cl, 13.50.

EXAMPLE 13

7-Fluoro-2-methoxy-3H-phenothiazin-3-one

Following the procedure described in Example 10 but substituting 2-amino-5-fluorothiophenol for 2-amino-3-methoxythiophenol and substituting 2-methoxy-p-benzoquinone for p-benzoquinone, the title compound was obtained, m.p. 252°C.

Analysis, calculated:

C, 59.76; H, 3.08; N, 5.36; S, 12.27; F, 7.26.

Observed: C, 59.60; H, 3.11; N, 5.20; S, 12.17; F, 7.33.

EXAMPLE 14

2,4-Dimethoxy-3H-phenothiazin-3-one

Following the procedure described in Example 10 but substituting 2-aminothiophenol for 2-amino-3-methoxythiophenol and substituting 2,6-dimethoxy-p-benzoquinone for p-benzoquinone, the title compound was obtained, m.p. 193°C.

Analysis, calculated:

C, 61.52; H, 4.06; N, 5.12; S, 11.73.

Observed: C, 61.37; H, 4.14; N, 5.16; S, 12.90.

## EXAMPLE 15

1,2-Dimethoxy-4-methyl-3H-phenothiazin-3-one

Following the procedure described in Example 10 but substituting 2-aminothiophenol for 2-amino-3-methoxythiophenol and substituting 2,3-dimethoxy-5-methyl-p-benzoquinone for p-benzoquinone, the title compound was obtained, m.p. 138°C.

Analysis, calculated:

C, 62.70; H, 4.56; N, 4.87; S, 11.16.

Observed: C, 62.72; H, 4.74; N, 4.92; S, 11.28.

## EXAMPLE 16

1,7-Dimethyl-3H-phenothiazin-3-one and 2,7-dimethyl-3H-phenothiazin-3-one

Following the procedure described in Example 10 but substituting 2-amino-5-methylthiophenol for 2-amino-3-methoxythiophenol and substituting 2-methyl-p-benzoquinone for p-benzoquinone, a mixture of the title compounds were obtained. Chromatography on silica gel eluting with 10% EtOAc in $CH_2Cl_2$ afforded firstly 2,7-dimethyl-3H-phenothiazin-3-one, m.p. 177°C.

Analysis, calculated:

C, 69.70; H, 4.60; N, 5.81; S, 13.29.

Observed: C, 69.51; H, 4.82; N, 5.78; S, 12,27, and secondly, 1,7-dimethyl-3H-phenothiazin-3-one. m.p. 168-170°C.

Analysis calculated:

C, 69.70; H, 4.60; N, 5.81; S, 13.29.

Observed: C, 69.59; H, 4.63; N, 5.80; S, 13.40.

EXAMPLE 17

2,4-Dichloro-7-fluoro-3H-phenothiazin-3-one

Following the procedure described in Example 10 but substituting 2-amino-5-fluorothiophenol for 2-amino-3-methoxythiophenol and substituting [illegible]-dichloro-p-benzoquinone for p-benzoquinone, the title compound was obtained, m.p. 256-258°C.

Analysis, calculated:

C, 48.02; H, 1.34; N, 4.68; S, 10.68; F, 6.33; Cl, 23.62.

Observed: C, 47.93; H, 1.42; N, 4.63; S, 10.75; F, 6.42; Cl, 23.80.

EXAMPLE 18

[illegible]Dichloro-7-fluoro-3H-phenothiazin-3-one

Following the procedure described in Example 10 but substituting 2-amino-5-fluorothiophenol for 2-amino-3-methoxythiophenol and substituting 2,5-dichloro-p-benzoquinone for p-benzoquinone, the title compound was obtained, m.p. 245-247°C.

Analysis, calculated:

C, 48.02; H, 1.34; N, 6.33.

Observed: C, 48.20; H, 1.14; N, 6.20.

EXAMPLE 19

2-Methoxy-7-methylthio-3H-phenothiazin-3-one

Following the procedure described in Example 10 but substituting 2-amino-5-methylthio thiophenol for 2-amino-3-methoxythiophenol and substituting 2-methoxy-p-benzoquinone for p-benzoquinone, the title compound was obtained, m.p. 222-224°C.

Analysis, calculated:

C, 58.11; H, 3.83; N, 4.84; S, 22.16.

Observed: C, 58.28; H, 4.24; N, 4.62; S, 22.02.

## EXAMPLE 20

4-Trifluoromethyl-3H-phenothiazin-3-one and 2,4-bis-(trifluoromethyl)-3H-phenothiazin-3-one

A solution of 3H-phenothiazin-3-one (10 g), trifluoromethyl iodide (50 g) and pyridine (40 ml) in acetonitrile (140 ml) was irradiated with a 450 watt lamp for 3 days. The volatiles were removed under vacuum and the resulting residue chromatographed on a silica gel golumn eluting with 5% EtOAc/$CH_2Cl_2$ to afford firstly, 2,4-bis(trifluromethyl)-3H-phenothiazin-3-one (650 mg) m.p. 173-175°C.

Analysis, calculated:
C, 48.14; H, 1.44; N, 4.01; S, 9.18; F, 32.64.
Observed: C, 48.25; H, 1.72; N, 4.00; S, 9.28; F, 32.51.

Secondly, 4-trifluoromethyl-3H-phenothiazin-3-one (1.76 g), m.p. 184-185°C.

Analysis, calculated:
C, 55.51; H, 2.15; N, 4.98; S, 11.40; F, 20.27.
Observed: C, 55.60; H, 2.14; N, 5.22; S, 11.43; F, 20.41.

## EXAMPLE 21

4-Acetyl-3H-phenothiazin-3-one

A solution of 3H-phenothiazin-3-one (2 g) and acetaldehyde (32 ml) in benzene (240 ml) was irradiated with a 450 watt lamp for 2 days. The volatiles were removed under vacuum and the residue chromatographed on a silica gel column eluting with 25% EtOAc/hexane to afford the desired compound, m.p. 222°C.

Analysis, calculated:

C, 65.87; H, 3.55; N, 5.49; S, 12.56.

Observed: C, 65.88; H, 3.61; N, 5.30; S, 12.70.

EXAMPLE 22

4-Bromo-2,7-dimethoxy-3H-phenothiazin-3-one

Step 1: 2-Methoxy-p-benzoquinone

Vanillin (2.432 kg) was added to a solution of sodium hydroxide (640 g) in water (8 l) and cooled to 10°C with an ice-bath. Then a soluton of hydrogen peroxide (30%) (2.4 l) was added at a rate to keep the temperature of the reaction mixture below 30°C. The addition completed (about 2 hours), the reaction mixture was added over a period of 3 hours to a suspension of sodium periodate (880 g) in water (4 l) and acetic acid (640 ml) cooled with an ice-bath to 10°C (the temperature of the reacton mixture was kept below 35°C). The precipitate was filtered, washed with cold water followed by ethanol/hexane (1:1) mixture and air-dried to afford the title compound (1.9 kg), m.p. 144-147°C.

Step 2: 2-Amino-5-methoxythiophenol

To a soluton of potassium hydroxide 8N (1.3 l) was added 2-amino-6-methoxybenzothiazole (750 g) and the mixture was refluxed for 18 hours. The resulting solution was neutralized by the addition of concentrated HCl, to pH 8.0, then acetic acid to pH 6.0. The precipitate which formed was filtered and washed with water to afford the title compound which was used immediately in Step 3.

Step 3: 2,7-Dimethoxy-3H-phenothiazin-3-one

To a suspension of 2-methoxy-p-benzoquinone, (1.15 kg) (Step 1) in methanol (8 l) was added portionwise a suspension of 2-amino-5-methoxythiophenol (from Step 2) in methanol (6 l). The reaction mixture was stirred for 15 minutes at room temperature, filtered and the collected solid washed with methanol (8 l). The product isolated was swished with DMF (16 l) for 2 hours, filtered and air-dried. The crude material was dissolved in hot DMF (16 l) (130°-140°C), filtered through Celite and the filtrate cooled to room temperature. The crystals were filtered, washed with methanol (8 l) and air-dried to afford the title compound (703 g), m.p. 237-238°C.

Step 4: 4-Bromo-2,7-dimethoxy-3H-phenothiazin-3-one

A solution of bromine (280 g) in acetic acid (2.8 l) was added over a period of 30 minutes to a suspension of 2,7-dimethoxy-3H-phenothiazin-3-one (250 g) (Step 3) in acetic acid (7.5 l) and stirred for 2 hours. Methanol (12 l) was added and the mixture was stirred until the black suspension became an orange suspension. Then, the precipitate was filtered, washed with methanol and air-dried to afford the desired compound (312 g), m.p. 260-261°C.

Analysis, calculated:

C, 47.74; H, 2.86; N, 3.98; S, 9.10; Br, 22.69.

Observed: C, 47.74; H, 2.81; N, 3.90; S, 9.02; Br, 22.37.

## EXAMPLE 23

4-Chloro-2-ethoxy-3H-phenothiazin-3-one and 4-chloro-2,7-diethoxy-3H-phenothiazin-3-one

Metallic sodium (506 mg) was dissolved in absorbed ethanol (75 ml) and 4-chloro-3H-phenothiazin-3-one (4.95 g) was added and stirred overnight at room temperature. The solvent was removed in vacuo, the resulting residue stirred in acetone (500 ml) for 1 hour and filtered. The filtrate was evaporated to dryness and the residue was chromatographed on a silica gel column eluting with 5% EtOAc/toluene to afford firstly, 4-chloro-2-ethoxy-3H-phenothiazin-3-one (1.33 g), m.p. 188-189°C.

Analysis, calculated:
C, 57.63; H, 3.45; N, 4.80; S, 10.99; Cl, 12.15.
Observed: C, 57.66; H, 3.54; N, 4.81; S, 11.16; Cl, 12.02.

and secondly, 4-chloro-2,7-diethoxy-3H-phenothiazin-3-one (110 mg), m.p. 227-228°C.

Analysis, calculated:
C, 57.22; H, 4.20; N, 4.17; S, 9.55; Cl, 10.56.
Observed: C, 57.19; H, 4.35; N, 4.07; S, 9.62; Cl, 10.61.

## EXAMPLE 24

2-(n-Butylthio)-3H-phenothiazin-3-one

To a solution of 3H-phenothiazin-3-one (0.64 g) in 75 ml methanol was added thiethylamine (1.0 ml) and n-butanethiol (0.58 ml). The mixture was stirred at room temperature for 48 hours. Then 2,3-dichloro-5,6-dicyano-1,4-benzoquinone (0.67 g) was added. The

mixture was stirred at room temperature for 2 hours. The solvent was removed *in vacuo*. The residue was chromatographed on neutral alumina (Act III) and eluted with 15% EtOAc/hexane to afford the title compound (0.4 g), m.p. 133°C.

Analysis, calculated:

C, 63.78; H, 5.02; N, 4.65

Observed: C, 63.61; H, 5.04; N, 4.51.

## EXAMPLE 25

4-(n-Butylthio)-3H-phenothiazin-3-one

To a solution of phenothiazin-3-one (0.21 g) in 20 ml THF was added triethylamine (0.28 ml) and n-butanethiol (0.2 ml). The mixture was refluxed for 15 hours and then cooled to room temperature. 2,3-Dichloro-5,6-dicyano-1,4-benzoquinone (DDQ) (0.22 g) was added and the reaction was stirred for 2 hours at 25°. The solvent was removed *in vacuo*. The residue was chromatographed on neutral alumina (Act III) and eluted with 15% EtOAc/hexane to afford the title compound, m.p. 72°C.

Analysis, calculated:

C, 63.78; H, 5.02; N, 4.05; S, 21.24.

Observed: C, 63.83; H, 5.07; N, 4.86; S, 21.06.

## EXAMPLE 26

4-(n-Butylthio)-2-methyl-3H-phenothiazin-3-one

To a solution of 2-methyl-phenothiazin-3-one (0.23 g) in 12 ml dichloroethane was added trie·hylamine (0.8 ml) and n-butanethiol (0.7 ml). The mixture was stirred at room temperature for 72 hours. 2,3-Dichloro-5,6-dicyano-1,4-benzoquinone (0.22 g) was added. The mixture was stirred at room

temperature for 2 hours. The mixture was stirred at room temperature for 2 hours. The solvent was removed in vacuo and the residue was chromatographed on neutral alumina (Act III) eluting with 15% EtOAc/hexane to give the title compound (120 mg), m.p. 97°C.

Analysis, calculated:

C, 64.75; H, 5.43; N, 4.44.

Observed: C, 64.62; H, 5.53; N, 4.43.

EXAMPLE 27

2-S-Glutathionyl-3H-phenothiazin-3-one

A mixture of phenothiazin-3-one (0.22 g), triethylamine (0.41 ml) and glutathione (0.3 g) in 1,2-dichloroethane (12 ml) was stirred at room temperature for 5 days. The solvent was removed in vacuo. The residue was dissolved in water and filtered. The filtrate was concentrated in vacuo and then chromatographed on XAD resin and eluted with water to give the title compound.

EXAMPLE 28

4-Chloro-2-S-glutathionylphenothiazin-3-one

Following the procedure of Example 27, but substituting 4-chloro-3H-phenothiazine-3-one for 3H-phenothiazin-3-one, the title compound was obtained.

EXAMPLE 29

5H-Benzo[a]phenothiazin-5-one

Following the procedure described in Example 10 but substituting 2-aminothiophenyl for 2-amino-3-methoxythiophenyl and substituting 1,4-naphthoquinone for p-benzoquinone the title compound was obtained, m.p. 176-177°C.

EXAMPLE 30

6-Chloro-5H-benzo[a]phenothiazin-5-one

Following the procedure described in Example 2, but substituting 5H-benzo[a]phenothiazin-5-one for 3H-phenothiazin-3-one, the title compound was obtained, m.p. 230-231°C.

EXAMPLE 31

6-Methyl-5H-benzo[a]phenothiazin-5-one

Following the procedure described in Example 10, but substituting 2-aminothiophenol for 2-amino-3-methoxythiophenol and substituting 2-methyl-1,4-naphthoquinone for p-benzoquinone, the title compound was obtained, m.p. 181°C.

Analysis, calculated:

C, 73.62; H, 4.00; N, 5.05; S, 11.56.

Observed: C, 73.77; H, 4.16; N, 4.99; S, 11.69.

EXAMPLE 32

1-Hydroxy-6-methyl-5H-phenothiazin-5-one

Following the procedure described in Example 10 but substituting 2-aminothiophenol for 2-amino-3-methoxythiophenol and substituting 5-hydroxy-2-methyl-1,4-naphthoquinone for p-benzoquinone, the title compound was obtained, m.p. 226-228°C.

Analysis, calculated:

C, 69.61; H, 3.78; N, 4.77; S, 10.93.

Observed: C, 69.66; H, 3.90; N, 4.66; S, 10.77.

EXAMPLE 33

1-Methoxy-6-methyl-5H-phenothiazin-5-one

Potassium tert.-butoxide (500 mg) was added to a suspension of 1-hydroxy-6-methyl-5H-benzo[a]-phenothiazin-5-one (from Example 32) (500 mg) and methyl iodide (2 ml) in DMF (20 ml). After 30 minutes at room temperature, EtOAc (250 ml) was added followed by water (200 ml). The aqueous layer was decanted and the organic layer was dried and evaporated to dryness. The residue was treated with ether, filtered and air-dried to afford the desired product (420 mg), m.p. 170-171°C.

Analysis, calculated:

C, 70.34; H, 4.26; N, 4.56; S, 10.43.

Observed: C, 70.37; H, 4.44; N, 4.45; S, 10.52.

EXAMPLE 34

4-Hydroxy-3H-phenothiazin-3-one-5,5-dioxide

To a suspension of 3-hydroxy-10H-phenothiazine-5,5-dioxide (1.75 g, 7 mmoles) in 2% aqueous sulfuric acid (25 ml) there was added, at room temperature, a solution of 80% sodium chlorite (3.17 g, 28 mmoles) in water (25 ml). The mixture was stirred for 15 minutes, then the red-organge precipitate was filtered to afford crude product (1.73 g). Purification was achieved by crystallization from DMF-methanol, m.p. 266° (dec.).

Analysis, calculated:

C, 55.16; H, 2.70; N, 5.36; S, 12.27.

Observed: C, 54.68; H, 2.76; N, 5.38; S, 12.47.

EXAMPLE 35

4-Chloro-3H-phenoxazin-3-one

To a solution of 1.2 g of 3H-phenoxazin-3-one in acetic acid (25 ml) was added $K_2Cr_2O_7$ (3.7 g). A solution of chlorine in acetic acid was added dropwise to the resulting suspension. After disappearance of the starting material, as monitored by TLC, the reaction mixture was poured into 200 ml of $H_2O$ and the resulting precipitate was filtered (1.2 g) and chromatographed on silica gel to yield the title compound.

Analysis, calculated:

C, 62.22; H, 2.61; Cl, 15.30.

Observed: C, 62.10; H, 2.75; Cl, 15.24.

EXAMPLE 36

2,4-Di-t-butyl-1H-phenothiazin-1-one

To a solution of 4.4 gm of 3,5-di-t-butyl-1,2-benzoquinone in 20 ml of ether was added a solution of 1.25 g of 2-aminothiophenol in 5 ml of ether. After stirring for 1 hour at 25°, the reaction mixture was evaporated. The residue was purified by flash chromatography on silica gel using 2% ethyl acetate in benzene as eluent. There was thus obtained 860 mg of the title compound as dark blue plates, m.p. 137-141°.

Analysis, calculated:

C, 73.81; H, 7.12; N, 4.30; S, 9.85

Observed: C, 73.77; H, 7.33; N, 4.33; S, 9.85.

## EXAMPLE 37

4-Bromo-1,7-dimethoxy-3-H-phenothiazin-3-one

To a suspension of 1,7-dimethoxy-3H-phenothiazin-3-one (300 mg) in acetic acid (9 ml) was added a 0.63 M solution of $Br_2$ in acetic acid (1.92 ml). After 15 minutes, methanol was added and the solid filtered, washed with ether and air dried to afford the title compound (353 mg), m.p. 267-270°C (dec).

## EXAMPLE 38

4-Chloro-1,7-dimethoxy-3H-phenothiazin-3-one and 2,4-dichloro-1,7-dimethoxy-2-H-phenothiazin-3-one

To a suspension of 1,7-dimethoxy-3H-phenothiazin-3-one (800 mg) in acetic acid (24 ml) was added a 1.15 M solution of $Cl_2$ in acetic acid (3.1 ml). After 15 minutes, methanol was added and the mixture was filtered, washed with ether and air dried to afford a mixture of the two title compounds (700 mg), which were separated on a silica gel column ($EtOAc:CH_2Cl_2$, 1:9), affording 4-chloro-1,7-dimethoxy-2H-phenothiazin-3-one, m.p. 278-280°C (dec.)

Analysis, Calculated:

C, 54.64; H, 3.28; N, 4.55; S, 10.42; Cl, 11.52.

Observed: C, 54.44; H, 3.26; N, 4.62; S, 10.54; Cl, 11.48.

and 2,4-dichloro-1,7-dimethoxy-3H-phenothiazin-3-one, m.p. 259-260°C (dec.) m/e 341.

## EXAMPLE 39

7-Methoxy-2-(4-methylpiperazin-1-yl)-3H-phenothiazine-3-one

A mixture of 7-methoxy-3H-phenothiazin-3-one (1.2 g) and N-methyl piperazine HCl (3.4g) in DMF (20 ml) was heated at 100°C for 3 hours. Then $NaIO_4$ (1g) was added and the reaction mixture was heated at 100°C for 1 hour. Ice-water was added to the reaction mixture followed by ethyl acetate. The aqueous layer was decanted, filtered and the filtrate basified with $K_2CO_3$ and extracted with ethyl acetate. The organic layer was evaporated to dryness, the resulting residue was dissolved in $CH_2Cl_2$, dried and evaporated to dryness to afford the crude final product (1.2 g) which was purified by chromatography on silica gel column eluting with 10% $MEOH/CH_2Cl_2$ to give the title compound, m.p. 208-209°.

## EXAMPLE 40

4-Bromo-7-methoxy-2-(4-methylpiperazin-1-yl)-3H-phenothiazin-3-one

To a suspension of 7-methoxy-2-(4-methyl piperazin-1-yl)-3H-phenothiazin-3-one (500 mg) in acetic acid (10 ml) was added a solution of bromine in acetic acid (0.5 M) (6ml) and stirred for 5 minutes. Hexane (100 ml) was added and the resulting precipitate was filtered. The solid was suspended in a mixture of aqueous $K_2CO_3$ (50 ml), EtOAc (100 ml) and methanol (20 ml) and stirred for 15 minutes. After filtration and decantation, the organic layer was washed with brine, dried and evaporated to dryness to afford the title compound (190 mg) m.p. 209-210° (dec.).

EXAMPLE 41

4-Bromo-2,7-dimethoxy-3H-phenothiazin-3-one-5,5-dioxide

STEP 1: 4-Bromo-3-hydroxy-2,7-dimethoxy-10-H-phenothiazine

To a suspension of 4-bromo-2,7-dimethoxy-3H-phenothiazin-3-one (100 g) in a mixture of ethylacetate (2 l) and water (1 l) was added sodium hydrosulfite (200 g) in one batch with mechanical stirring. The orange reaction mixture was stirred for 15 hours under a nitrogen atmosphere. The resulting white reaction mixture was filtered and the precipitate washed with water under a nitrogen atmosphere to prevent air oxidation of the compound. The title compound (130 g) was obtained as a wet material and was used as such in the next step (Step 2). An analytical sample was air dried, m.p. 185°C.
Analysis, Calculated:
C, 47.47; H, 3.42; N, 3.95; S, 9.05; Br, 22.56.
Observed: C, 47.21; H, 3.39; N, 3.74; S, 8.76; Br, 22.44.

STEP 2: 3-Acetoxy-4-bromo-2,7-dimethoxy-10-H-phenothiazine

Wet 4-bromo-3-hydroxy-2,7-dimethoxy-10-H-phenothiazine (130 g) (from step 1) was suspended in pyridine (230 ml). The mixture was cooled to 0°C in an ice-water bath. Acetic anhydride (195 ml) was then slowly added. The solution was left stirring at room temperature for 1/2 hour. The mixture was then concentrated under reduced pressure

to approximately 1/3 of the original volume. The a mixture of ether:hexane, 1:1 (700 ml) was added, causing a large amount of crystals to appear. These crystals were filtered, washed with ether, and air dried, giving 51.4 g of pure 3-acetoxy-4-bromo-2,7-dimethoxy-10-phenothiazine. The mother liquors were reevaporated, and ether and hexane were added again, giving 36.37 of crude 3-acetoxy-4-bromo-2,7-dimethoxy-10H-phenothiazine m.p. 201-203°C.

Analysis, Calculated:

C, 48.50; H, 3.56; N, 3.53; S, 8.09.

Observed: C, 48.31; H, 3.47; N, 3.47; S, 8.00.

STEP 3: 3-Acetoxy-4-bromo-2,7-dimethoxy-10H-phenothiazin-5,5-dioxide

To 3-acetoxy-4-bromo-2,7-dimethoxy-10H-phenothiazin (20 g) (from Step 2) in suspension in $CH_2Cl_2$:MeOH, (1:1) (500 ml), was added m-chloroperoxybenzoic acid (26.0 g). The reaction mixture rapidly became deep brown with the formation of a yellowish precipitate which corresponded to the intermediate sulfoxide on the 5-position. The mixture was heated at reflux for 18 hours. The solid was then filtered and washed with ether. Since there was still some sulfoxide remaining, the solid was suspended in ethanol:1,2-dichloroethane (500 ml) with 1.35 g of m-chloroperoxybenzoic acid and heated at reflux overnight (15 hours). The solid was then filtered and washed with ether and air dried giving 13.0 g of 3-acetoxy-4-bromo-2,7-dimethoxy-10H-phenothiazine-5,5-dioxide, m.p. 260°C.

Analysis, Calculated:

C, 44.87; H, 3.29; N, 3.27; S, 7.49

Observed: C, 44.82; H, 3.21; N, 3.18; S, 7.67.

STEP 4: 4-Bromo-3-hydroxy-2,7-dimethoxy-10H-phenothiazin-5,5-dioxide

To a suspension of 3-acetoxy-4-bromo-2,7-dimethoxy-10H-phenothiazine-5,5-dioxide (10.0 g) in methanol (105 ml) was added a solution of 2N aqueous sodium hydroxide (74 ml) under a nitrogen atmosphere. After 20 minutes, the mixture was acidified with 10% v/v aqueous acetic acid (250 ml), causing a large amount of compound to precipitate. The mixture was then diluted with water (105 ml) and the solid filtered, washed with water and ether and dried in a dessicator to afford quantitatively the title compound, m.p. 252-260°C (dec).

STEP 5: 4-Bromo-2,7-dimethoxy-3H-phenothiazin-3-one-5,5-dioxide

To a stirred suspension of 4-bromo-3-hydroxy-2,7-dimethoxy-10H-phenothiazin-5,5-dioxide (from Step 4) (1 g) in THF (10 ml) was added 2,3-dichloro-5,6-dicyano-1,4-benzoquinone (1.17 g). After 15 minutes, the mixture was filtered, the solid washed with ether and air dried. The solid was filtered through a silica gel pad with $CH_2Cl_2$:EtOAc, 1:1, to afford the title compound (300 mg), m.p. 228-230°C (dec), m/e 383.

EXAMPLE 42

2,7-Dimethoxy-4-(4-methylpiperazin-1-yl)-3H-phenothazin-3-one-5,5-dioxide

To a stirred suspension of 4-bromo-3-hydroxy-2,7-dimethoxy-10H-phenothiazine-5,5-dioxide (1g) in THF (10 ml) was added 2,3-dichloro-5,6-dicyano-1,4-benzoquinone (1.17g). After a period of 20

minutes, N-methyl piperazine (1.44 ml) was slowly added. After 20 minutes, hexane was added to the mixture and the resulting precipitate was filtered, washed with ether and air dried. The compound was chromatographed using $CH_2Cl_2$:MeOH (9.5:0.5) as eluant, to afford the title compound (242 mg), m.p. 261°C (dec.)

Analysis; Calculated:

C,58.67; H.5.83; N, 10.80; S, 8.24

Observed: C, 58.73; H,5.67; N, 10.83; S,8.56

## EXAMPLE 43

4-Hydroxy-2,7-dimethoxy-3H-phenothiazin-3-one-5,5-dioxide

To a stirred suspension of 4-bromo-3-hydroxy-2,7-dimethoxy-10H-phenothiazine-5,5-dioxide (100 mg) in THF (10 ml) was added water (0.1 ml) and 2,3-dichloro-5,6-dicyano-1,4-benzoquinone (0.12 g). After 20 minutes, hexane was added and the solid was filtered, washed with ether and air dried to afford the title compound, m.p. 333-335°C (dec.)

## EXAMPLE 44

1,4-Bis(1-propylamino)-3H-phenothiazin-3-one 5,5-dioxide

To a solution of 3-hydroxy-10H-phenothiazine-5,5-dioxide (989 mg) in THF (50 ml) was added 2,3-dichloro-5,6-dicyano-1,4-benzoquinone (1.82 g). The resulting green mixture was stirred at room temperature for 3 minutes, then there was added n-propyl amine (2.36 g). The mixture was stirred for 20 minutes and then filtered. The filtrate was evaporated to dryness and the residue chromatographed

on a column of silica gel eluting with a 1:20 mixture of ethyl acetate and dichloromethane to afford the title compound as a purple solid (850 mg). Crystallization from methane afforded purple crystals (606mg), m.p. 174-176°C

Analysis, Calc'd:

C, 60.14; H, 5.89; N, 11.69; S, 8.92.

Found: C, 60.08, H, 5.93; N, 11.80; S, 8.71.

EXAMPLE 45

1,4-Bis(4-methylpiperazin-1-yl)-3H-phenothiazine-3-one-5,5-dioxide

The procedure of Example 44 was used, substituting N-methyl piperazine for n-propyl amine to afford the title compound. It was crystallized from a toluene-hexane mixture to afford red crystals, mp: 247-249°C (dec).

Analysis, Calc'd:

C, 59.84; H, 6.16; N, 15.86; S, 7.26

Found: C, 59.98, H, 6.35; N, 15.58; S, 7.1.

EXAMPLE 46

6-(1-Propylamino)-5H-benzo[a]phenothiazin-5-one-7,7-dioxide

STEP 1: 5-acetoxy-12H-benzo[a]phenothiazine

To a stirred solution of 5-hydroxy-12H-benzo[a]phenothiazine (50 g) in pyridine (115 ml) was added acetic anhydride (48ml). The reaction was exothemic stirring was continued without cooling for 30 minutes, then the mixture was cooled to 10°C using an ice bath. The yellow crystalline solid was

filtered and washed with ether to afford the title compound (23.4 g). The product was crystallized from etyl acetate, m.p. 185-186°C.

STEP 2: 5-acetoxy-12H-benzo[a]phenothiazine-7,7-dioxide

To a suspension of 5-acetoxy-12H-benzo[a] phenothiazine (10 g) in dichloromethane (125 ml), was added a solution of m-chloroperoxybenzoic acid (18 g) in methanol (125 ml). The mixture was refluxed for 2.5 hours, then after cooling to room temperature the insoluble solid was filtered to afford the desired sulfone (8.6 g). The solid was recrystallized from THF m.p. 284-287°C

Analysis, Calc'd:

C, 63.70; H, 3.86; N, 4.13; S, 9.45.

Found: C, 63.67; H, 3.82; N, 4.20; S, 9.44

STEP 3: 5-hydroxy-12H-benzo[a]phenothiazine-7,7 dioxide

To a suspension of 5-acetoxy-12H-benzo[a] phenothiazine-7,7-dioxide (6.6 g) in methanol (200 ml), kept under a nitrogen atmosphere was added 2N aqueous sodium hydroxide solution (132 ml). The mixture was stirred at room temperature for 7 minutes, then there was added 10% acetic acid (200 ml) and water (300 ml). After 10 minutes of stirring the mixture was filtered to afford the title compound (5.68 g) as a pink solid. The solid was recrystallized from THF, m.p. 334°C (dec).

Analysis, Calc'd:

C, 63.70, H, 3.86; N, 4.13; S, 9.45.

Found: C, 63.67, H, 3.82; N, 4.20; S, 9.44.

STEP 4 6-(1-Propyl amino)-5H-benzo [a] phenothiazin-5-one-7,7-dioxide

To a suspension of 5-hydroxy-12H-benzo[a] phenothiazine-7,7-dioxide (594mg) in THF (10 ml) was added 2,3-dichloro 5,6-dicyano-1,4-benzooquinone (1.021 gram). The mixture was stirred at room temperature for 2 minutes, then there was added n-propyl amine (0.2ml). The stirring was continued for 0.5 hour, then the mixture was evaporated to dryness. To the residue was added 50 ml dichloromethane and the mixture was stirred for 15 minutes and filtered. The filtate was evaporated to dryness and the residue crystallized from a mixture of toluene and hexane to afford the title compound (442 mg) as a red-brown crystalline solid, m.p. 149-151°C (dec.).

Analysis, Calc'd:

C, 64.75; H, 4.58; N, 7.95; S, 9.10.

Found: C, 64.66, H, 4.48; N, 7.95; S, 9.04.

EXAMPLE 47

6-(4-Methyl piperazin-1-yl)-5H-benzo[a]phenothiazin-5-one-7,7-dioxide

The procedure of Example 46, Step 4 was used, substituting N-methyl piperazine for n-propyl amine, to afford the title compound, m.p. slow dec. from 183° C.

Analysis, Calc'd:

C, 64.10; H, 4.87; N, 10.68; S, 8.15.

Found: C, 63.89, H, 4.90; N, 10.56; S, 8.10.

EXAMPLE 48

6-Amino-5H-benzo[a]phenothiazin-5-one-7,7-dioxide

The procedure of Example 46, Step 4 was used, substituting 28% aqueous ammonium hydroxide solution for n-propyl amine, to afford the title compound, m.p. 264-266°C.

Certain of the compounds herein disclosed contain one or more centers of asymmetry. The present invention is meant to include the various diastereomers of such compounds as well as their racemic and optically active resolved forms.

Some of the compounds described may exist in one or more tautomeric forms. All such tautomeric forms are included within the present invention.

EXAMPLE 49

TEST RESULTS

Following the procedure of assay B (Indomethacin-Induced Ulcer Assay), 4-bromo-2,7-dimethoxy-3H-phenothiazin-3-one was tested for ulcer inhibition. For Table 49-1, % Inhibition was calculated as:

$$\frac{\text{Mean Ulcers (Control)} - \text{Mean Ulcers (Test)}}{\text{Mean Ulcers (Control)}} \times 100 = \text{\% Inh.}$$

TABLE 49-1

INDOMETHACIN-INDUCED ULCER ASSAY

| Dose (mg/kg) | % Inhibition |
|---|---|
| 0.003 | 38 |
| 0.01 | 56 |
| 0.03 | 69 |
| 0.1 | 66 |
| 0.3 | 70 |
| 3.0 | 87 |

CLAIMS

1. Use of a lipoxygenase inhibitor in a process for the production of a cytoprotective pharmaceutical composition which contains as cytoprotective active ingredient at least one lipoxygenase inhibitor.

2. Use according to patent claim 1, characterized in that the used lipoxygenase inhibitor is a compound of formula I

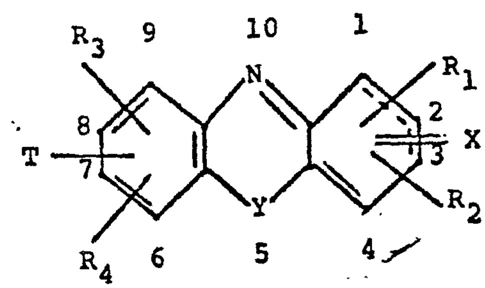

I

wherein

X is in the 1 or 3 position and is O, S or NR;

R is H, $C_1$-$C_6$ branched or linear alkyl, CN or phenyl;

Y is O, Se, S, SO, $SO_2$ or NR; and the broken line represents an optional double bond between the 1 and 2 or 2 and 3 position;

$R_1$, $R_2$, $R_3$ and $R_4$ are each independently selected from:

(1) hydrogen

(2) alkyl having 1-6 carbon atoms,

(3) alkenyl having 2-6 carbon atoms,

(4) $-(CH_2)_n M$

wherein n is 0-6 and

M is a) $OR_5$,

b) halogen,

c) $CF_3$,

d) $SR_5$ wherein $R_5$ is H; lower alkoxyl-loweralkyl; lower acyloxy-loweralkyl; $C_1$-$C_6$ alkyl; benzyl; phenyl or substituted phenyl wherein the substituents are $C_1$-$C_3$ alkyl, halogen, CN, $CF_3$, $COOR_6$, $CH_2COOR_6$, $(CH_2)_nNR_8R_9$ wherein n is 0 to 2, $C_1$-$C_3$ alkoxy, OH, halo-$C_1$-$C_6$-alkyl; $-(CH_2)_mCOOR_6$, wherein m is 0 to 6 and $R_6$ is H, phenyl or $C_1$-$C_6$ alkyl; CN, formyl; perfluoroalkyl; or $CH_2$-$R_{12}$, wherein $R_{12}$ is $C_1$-$C_5$ alkyl, phenyl or dimethylamino;

e) phenyl or substituted phenyl as defined above for $R_5$;

f) $COOR_6$;

g) $\overset{O}{\overset{\|}{C}}-R_{14}$ wherein $R_{14}$ is H, $(CH_2)_n$ $COOR_6$ wherein n is 0 to 4, $C_1$-$C_6$ alkyl, $CF_3$, phenyl, or substituted phenyl as defined above for $R_5$;

h) tetrazole;

i) $-NH-\overset{O}{\overset{\|}{C}}-R_7$ wherein $R_7$ is $C_1$-$C_6$ alkyl benzyl or phenyl;

j) $-NR_8R_9$ wherein $R_8$ and $R_9$ are independently selected from H, phenyl or substituted phenyl as defined above for $R_5$ or $C_1$-$C_4$ alkyl, $C_1$-$C_4$ alkylamino alkyl, or may be joined through the N to form a heterocycloalkyl of 5-8 ring atoms;

k) $-NHSO_2R_{10}$ wherein $R_{10}$ is OH, $C_1$-$C_6$ alkyl, $C_1$-$C_6$-alkoxy, phenyl or $CF_3$;

l) $-\overset{O}{\overset{\|}{C}}-CH_2OH$;

m) $-SOR_{11}$ wherein $R_{11}$ is $C_1$-$C_6$ alkyl, phenyl or substituted phenyl as defined above for $R_5$, $(CH_2)_mCOOR_6$ wherein m is 1 to 6, CN, formyl or perfluoro-$C_1$-$C_4$ alkyl;

n) $-CONR_8R_9$

o) $-SO_2NR_8R_9$;

p) $-SO_2R_{13}$ wherein $R_{13}$ is OH, H, $C_1$-$C_6$-alkyl, phenyl or substituted phenyl as defined above for $R_5$,

$(CH_2)_mCOOR_6$ wherein m is 1 to 6, CN or perfluoro-$C_1$-$C_4$ alkyl;

q) $NO_2$;

r) $-O-\overset{O}{\overset{\|}{C}}-R_{14}$ ;

s) $-O-\overset{O}{\overset{\|}{C}}-NR_8R_9$ ;

t) $-O-\overset{O}{\overset{\|}{C}}-OR_7$

u) -CN ;

v) $NR_{15}$ $R_{16}$ wherein $R_{15}$ and $R_{16}$ are such that $HNR_{15}R_{16}$ is an essential amino acid; or

(5) any two of $R_1$, $R_2$, $R_3$ and $R_4$ may be joined to form a fourth ring, which may be saturated or unsaturated, of five or six carbons; and

T is H, halogen or $CF_3$

or a pharmaceutically acceptable salt of the compound of formula I and that the lipoxygenase inhibitor or the salt thereof is used to produce a cytoprotective pharmaceutical composition which optionally furthermore contains a pharmaceutically acceptable carrier.

3. Use according to patent claim 2, characterized in that the cytoprotective active ingredient of formula I is 4-bromo-2,7-dimethoxy-3H-phenothiazin-3-one or a salt thereof is used.

4. Use according to one of the patent claims 1 - 3, characterized in that the lipoxygenase inhibitor is used in an amount to produce a pharmaceutical composition with which the lipoxygenase inhibitor can be administered in an amount of 0,1 µg - 500 mg of the lipogygenase inhibitor per kg body weight, preferably in an amount of 1 µg to 50 mg lipoxygenase inhibitor per kg body weight.

5. Use according to one of the patent claims 1 - 4, characterized in that the lipoxygenase inhibitor is used for the production of a cytoprotective pharmaceutical composition, which comprises as further component at least one non-steroidal anti-inflammatory drug and that the used lipoxygenase inhibitor reduces the undesirable side effects of the non-steroidal anti-inflammatory drug.

6. Use according to patent claim 5, characterized in that the weight ratio of the used lipoxygenase inhibitor to the used non-steroidal anti-inflammatory drug in the so produced pharmaceutical composition is in the range of 1000:1 to 1:1000, preferably in the range of 200:1 to 1:200.

7. Process for the preparation of a cytoprotective pharmaceutical composition, characterized

in that into a pharmaceutical composition there is incorporated a cytoprotective effective amount of at least one lipoxygenase inhibitor.

8. Process according to patent claim 7, characterized in that the incorporated lipoxygenase inhibitor is a compound of formula I:

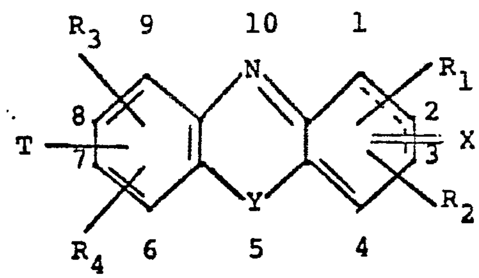

I

wherein

X is in the 1 or 3 position and is O, S or NR wherein R is H, $C_1$-$C_6$ branched or linear alkyl, CN or phenyl;
Y is O, Se, S, SO, $SO_2$ or NR; and the broken line represents an optional double bond between the 1 and 2 or 2 and 3 position;
$R_1$, $R_2$, $R_3$ and $R_4$ are independently selected from:

(1) hydrogen;
(2) alkyl having 1-6 carbon atoms;
(3) alkenyl having 2-6 carbon atoms;
(4) $-(CH_2)_nM$

wherein n is 0-6 and M is

a) $OR_5$;

b) halogen;

c) $CF_3$;

d) $SR_5$ wherein $R_5$ is H; lower alkoxy-lower alkyl; lower acyloxy-lower alkyl; $C_1$-$C_6$ alkyl; benzyl; phenyl or substituted phenyl wherein the substituents are $C_1$-$C_3$ alkyl, halogen, CN, $CF_3$, $COOR_6$, $CH_2COOR_6$, $(CH_2)_nNR_8R_9$ wherein n is 0 to 2, $C_1$-$C_3$ alkoxy, OH, halo-$C_1$-$C_6$-alkyl; $-(CH_2)_mCOOR_6$, wherein m is 0 to 6 and $R_6$ is H, phenyl, or $C_1$-$C_6$ alkyl; CN; formyl; perfluoroalkyl; or $CH_2$-$R_{12}$ wherein n is 0 to 4, $R_{12}$ is $C_1$-$C_5$ alkyl, dimethylamino or phenyl;

(e) phenyl or substituted phenyl as defined above for $R_5$;

(f) $COOR_6$;

(g) $\overset{\overset{O}{\|}}{C}-R_{14}$ wherein $R_{14}$ is H, $(CH_2)_nCOOR_6$ wherein n is 0 to 4, $C_1$-$C_6$ alkyl, $CF_3$, phenyl, or substituted phenyl as defined above for $R_5$;

(h) tetrazole;

(i) $-NH-\overset{\overset{O}{\|}}{C}-R_7$ wherein $R_7$ is $C_1$-$C_6$ alkyl, benzyl or phenyl;

(j) $-NR_8R_9$ wherein $R_8$ and $R_9$ are independently selected from H, phenyl or substituted phenyl as defined above for $R_5$, $C_1$-$C_4$ alkyl, $C_1$-$C_4$ alkylamino alkyl, or may be joined through the N to form a heterocycloalkyl of 5-8 ring atoms;

(k) $-NHSO_2R_{10}$ wherein $R_{10}$ is OH, $C_1$-$C_6$ alkyl, $C_1$-$C_6$-alkoxy, phenyl, or $CF_3$;

(l) $-\overset{\overset{O}{\|}}{C}-CH_2OH$;

(m) $-SOR_{11}$ wherein $R_{11}$ is $C_1$-$C_6$ alkyl, phenyl or substituted phenyl as defined

above for $R_5$, $(CH_2)_mCOOR_6$ wherein m is 1 to 6, CN, formyl or perfluoro-$C_1$-$C_4$ alkyl;

(n) $-CONR_8R_9$;

(o) $-SO_2NR_8R_9$;

(p) $-SO_2R_{13}$ wherein $R_{13}$ is OH, $C_1$-$C_6$ alkyl, H, phenyl or substituted phenyl as defined above for $R_5$, $(CH_2)_mCOOR_6$ wherein m is 1 to 6, CN, formyl or perfluoro-$C_1$-$C_4$ alkyl;

(q) $NO_2$ ;

(r) $O-\overset{O}{\overset{\|}{C}}-R_{14}$ ;

(s) $O-\overset{O}{\overset{\|}{C}}-NR_8R_9$

(t) $O-\overset{O}{\overset{\|}{C}}-OR_7$ ;

(u) CN;

(v) $NR_{15}R_{16}$ wherein $R_{15}$ and $R_{16}$ aare such that $HNR_{15}R_{16}$ is an essential amino acid; or

(5) any two of $R_1$, $R_2$, $R_3$ and $R_4$ may be fused to add a fourth ring, which may be saturated or unsaturated, of five or siv carbons; and

T is H, halogen or $CF_3$

or is a pharmaceutically acceptable salt of the compound of formula I.

9. Process according to patent claim 8, characterized in that the incorporated lipoxygenase inhibitor is 4-bromo-2,7-dimethoxy-3H-phenothiazin-3-one or a pharmaceutically acceptable salt thereof.

10. Process according to one of the patent claims 7 - 9, characterized in that a cytoprotective pharmaceutical composition is prepared, which contains in addition to the cytoprotective amount of the lipoxygenase inhibitor at least one further pharmacologically active ingredient, preferably a second component having cytoprotective activity, a non-steroidal anti-inflammatory drug, a prostaglandin antagonist, a thromboxane antagonist, a histidine decarboxyase inhibitor, a $H_1$ or $H_2$-receptor antagonist and/or a $K^+/H^+$ ATPase inhibitor.